# EUROPEAN PATENT APPLICATION

(11) **EP 4 471 022 A1**
(43) Date of publication of application: **04.12.2024**
(21) Application number: 23746267.6
(22) Date of filing: 19.01.2023
(51) Int. Cl.: C07D 401/04, C07D 405/02, A61K 31/4353, A61K 31/165, A61P 25/28

(54) **AMIDE COMPOUND AS POTASSIUM CHANNEL REGULATOR, AND PREPARATION THEREFOR AND USE THEREOF**

(30) Priority: 25.01.2022 CN 202210088634
(71) Applicant: Shanghai Zhimeng Biopharma, Inc., Shanghai 201210 (CN)
(72) Inventor: LIANG, Bo, Shanghai 201210 (CN); LIU, Gang, Shanghai 201210 (CN); JIANG, Zhaojian, Shanghai 201210 (CN); HUANG, Mengwei, Shanghai 201210 (CN); CHEN, Huanming, Shanghai 201210 (CN)
(74) Representative: Santoro, Sofia
(86) International application number: PCT/CN2023/073191
(87) International publication number: WO 2023/143388

(57) **Abstract**

Provided are an amide compound as a potassium channel regulator, and the preparation therefor and the use thereof. The compound has a structure as represented by formula I, wherein the definition of each group and substituent is as described in the description.

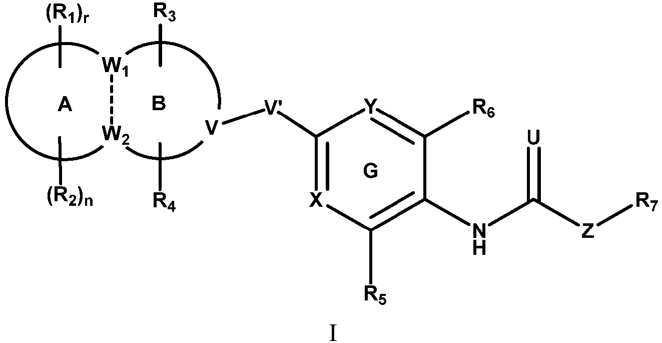

## Description

### Technical field

The present invention relates to biomedical field, specifically to an amide compound as potassium channel regulator, and preparation therefor and use thereof.

### Background

Kv7 potassium channel is a class of voltage dependent potassium ion channels, characterized by low threshold activation, slow activation, and non deactivation. The Kv7 potassium channel has five family members (Kv7.1-Kv7.5), and all Kv7 potassium channel members have similar topological structures, i.e., four subunits form a functional channel, with each subunit containing six transmembrane segments (S1-S6). Among them, S4 is the voltage sensing region, which plays an important role in sensing membrane potential changes and controlling conformational changes; SS-S6 is the main component of the channel pore area and the main combination and action area of potassium channel opener. KV7.1 potassium channel is a non neuronal pathway distributed in peripheral tissues and expressed in the heart to mediate myocardial Iks, and the mutations of which can lead to Long Q-T syndrome. Kv7.2-Kv7.5 potassium channels are the basis of neuronal M currents, widely distributed in the nervous system, and have various physiological activities. Mutations in the Kv7.2 and Kv7.3 potassium channel genes can lead to various epilepsy phenotypes, such as benign familial neonatal convulsions (BFNC), which fully demonstrate the role of M currents in regulating neuronal excitability. Kv7.4 potassium channel is highly expressed in the outer hair cells of the cochlea and brainstem auditory nucleus, and its mutations may lead to hereditary hearing loss. Kv7.5 potassium channel is highly expressed in skeletal muscle and brain, and its mutations may lead to retinopathy. Many diseases such as epilepsy, anxiety, and deafness share the common feature of high membrane excitability, and Kv7 potassium channels, as the molecular basis for M-currents, can be opened by sensing changes in membrane potential, and upregulate inhibitory potassium currents, thereby controlling membrane excitability, making Kv7 potassium channels of great significance in pain and mental disorders represented by high neural excitability.

Retigabine is a drug used to treat epilepsy and has been approved for marketing in UK, Germany, and Denmark. Research has confirmed that the action of Ritegabine is related to voltage gated potassium ion channels (KCNQs), with its main mechanism of action being the regulation of M-type potassium currents by KCNQ2/3 channels.

Ritegabine (RTG) was the first Kv7 potassium channel opener launched in 2011 as an adjuvant therapy for adult partial seizure epilepsy. In addition to anti-epileptic effects, RTG can also be used to treat anxiety disorders, neuralgia, neurodegenerative diseases, etc. RTG can effectively reduce or prevent epileptic seizures in various epilepsy models. RTG has shown effective anti epileptic effects on both tonic seizures induced by the Maximum Electroshock (MES) model and clonic seizures induced by PTZ. In addition, RTG can also prevent epileptic seizures caused by N-methyl-D-aspartate (NMDA), penicillin, picrotoxin, kainic acid (KA), etc. The ignition model is suitable for screening various anti-epileptic drugs, and the effect of RTG on this model is stronger than on other models. Due to the extensive effects of RTG on all Kv7 potassium channel members and other channels, its selectivity is poor, making it potentially harmful. A large number of literatures have reported that RTG has a high incidence of adverse events related to the central nervous system, which can lead to dizziness, fatigue, aphasia, speech disorders, balance disorders and other adverse reactions, including kidney stones, urinary retention and other kidney and urinary system diseases, cardiac arrest, transient non persistent ventricular tachycardia and other heart related diseases, and can also lead to retinal discoloration, skin, nails and other blue/purple pigmentation.

### Summary

The purpose of the present invention is to provide a compound shown in formula I and preparation method therefor, and the use as a potassium channel regulator.

In the first aspect of the present invention, provided is a compound of formula I, or a pharmaceutically acceptable salt thereof, 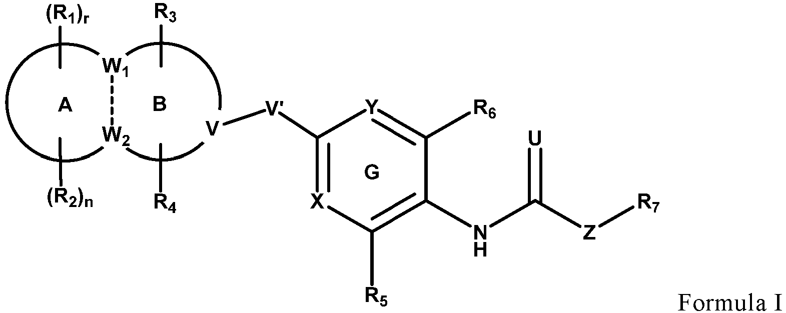 wherein,
ring A is selected from the group consisting of: none, C₆₋₁₀ aryl, 4-7-membered heteroaryl containing 1-3 heteroatoms selected from N, O and S, saturated or unsaturated C₃₋₆ cyclic hydrocarbon group, and 3-10-membered heterocyclyl containing 1-3 heteroatoms selected from N, O and S;
R₁, R₂, R₃ and R₄ are independently selected from the substituted or unsubstituted group consisting of: hydrogen, deuterium, halogen, cyano, -OH, -COOH, nitro, C₁₋₆ alkyl, C₃₋₆ cycloalkyl, C₁₋₆ alkoxy, C₃₋₆ cycloalkyloxy, C₂₋₆ alkenyl, C₂₋₆ alkynyl, saturated or unsaturated C₃₋₆ cyclic hydrocarbon group, 3-10-membered heterocyclyl containing 1-3 heteroatoms selected from N, O and S, C₆₋₁₀ aryl, 5-14-membered heteroaryl containing 1-3 heteroatoms selected from N, O and S, C₆₋₁₂ arylalkyl, -N(R₁')(R₂'), -C(O)-R₁', -C(O)-N(R₁')(R₂'), -C(O)-OR₁', -N(R₁')-C(O)-R₂', -S(O)ₘ-R₁', -S(O)ₘ-N(R₁')(R₂'), -S(O)ₘ-OR₁', -N(R₁')-S(O)ₘ-R₂', and the "substituted" refers to being substituted by one or more substituents selected from the group consisting of: halogen, C₁₋₆ alkyl, C₃₋₆ cycloalkyl, C₁₋₆ alkoxy, C₃₋₆ cycloalkyloxy, C₁₋₆ haloalkyl, C₃₋₆ halocycloalkyl, C₁₋₆ haloalkoxy and C₃₋₆ halocycloalkyloxy;
n and r are independently selected from the group consisting of: 0, 1 and 2;
R₁' and R₂' are independently selected from the group consisting of: hydrogen, C₁₋₆ alkyl, and C₃₋₆ cycloalkyl, or R₁' and R₂' together with the attached N-atom form a saturated or unsaturated 3-10-membered heterocyclyl containing 1-3 heteroatoms selected from N, O and S; and the above alkyl, cycloalkyl and heterocyclyl are optionally substituted by one or more substituents selected from the group consisting of: =O, halogen, C₁₋₆ alkyl and C₃₋₆ cycloalkyl;
m is selected from the group consisting of: 1 and 2;
W₁ and W₂ are independently selected from the group consisting of: none, C and N; and W₁ and W₂ are not simultaneously none;
------ is selected from the group consisting of: none, single bond and double bond; ring B is selected from the group consisting of: C₆₋₁₀ aryl, 4-7-membered heteroaryl containing 1-3 heteroatoms selected from N, O and S, saturated or unsaturated C₃₋₁₀ cyclic hydrocarbon group, 3-10-membered heterocyclyl containing 1-3 heteroatoms selected from N, O and S, 3-10-membered bridged heterocyclyl containing 1-3 heteroatoms selected from N, O and S;
V is selected from the group consisting of: C, CRs and N;
R₈ is selected from the group consisting of: hydrogen, C₁₋₆ alkyl, C₃₋₆ cycloalkyl, C₂₋₆ alkenyl and C₂₋₆ alkynyl; and the above alkyl and cycloalkyl are optionally substituted by one or more substituents selected from the group consisting of halogen, C₁₋₆ alkyl and C₃₋₆ cycloalkyl; V' is selected from the group consisting of: -(CH₂)ₚ-, -C(CH₃)₂-, 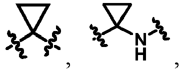 -NH-, -(CH₂)ₚ-NH-, -CF₂-NH-, - C(CH₃)₂-NH- and 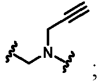
p is selected from the group consisting of: 0, 1 and 2;
X and Y are independently selected from the group consisting of: N and CR₉;
R₉ is selected from the group consisting of: hydrogen, halogen, cyano, amino, hydroxyl, C₁₋₆ alkyl, C₃₋₆ cycloalkyl and C₁₋₆ alkoxy; and the above amino, alkyl, cycloalkyl and alkoxy are optionally substituted by one or more substituents selected from the group consisting of halogen, C₁₋₆ alkyl, and C₃₋₆ cycloalkyl;
R₅ and R₆ are independently selected from the group consisting of: hydrogen, halogen, cyano, amino, hydroxyl, C₁₋₆ alkyl, C₃₋₆ cycloalkyl and C₁₋₆ alkoxy; and the above amino, alkyl, cycloalkyl and alkoxy are optionally substituted by one or more substituents selected from the group consisting of halogen, C₁₋₆ alkyl and C₃₋₆ cycloalkyl;
U is selected from the group consisting of: O, S and N(R₁₀);
Z is selected from the group consisting of: O, -(CH₂)_{q}- and -N(R₁₁)-;
q is selected from the group consisting of: 0, 1 and 2;
R₁₀ and R₁₁ are independently selected from the group consisting of: hydrogen, C₁₋₆ alkyl and C₃₋₆ cycloalkyl; and the above alkyl and cycloalkyl are optionally substituted by one or more substituents selected from the group consisting of halogen, C₁₋₆ alkyl and C₃₋₆ cycloalkyl;
R₇ is selected from the group consisting of: C₁₋₆ alkyl, C₃₋₆ cycloalkyl, C₅₋₈ bridged cyclic group, adamantyl, C₆₋₁₀ aryl, 3-10-membered heteroaryl containing 1-3 heteroatoms selected from N, O and S, 4-8-membered heterocycloalkyl containing 1-3 heteroatoms selected from N, O and S, C₃₋₆ cycloalkenyl, C₂₋₆ alkenyl and C₂₋₆ alkynyl; and the above alkyl, cycloalkyl, bridged cyclic group, adamantyl, aryl, heteroaryl, heterocycloalkyl, cycloalkenyl, alkenyl, and alkynyl are optionally substituted by one or more substituents selected from the group consisting of: hydrogen, halogen, cyano, nitro, amino, hydroxyl, C₁₋₆ alkyl-CO-, C₁₋₆ alkyl, C₃₋₆ cycloalkyl, C₆₋₁₀ aryl, C₁₋₆ haloalkyl, C₁₋₆ alkoxy, C₁₋₆ alkylamino and C₁₋₆ haloalkoxy.

In another preferred embodiment,
ring A is selected from the group consisting of: none, C₆₋₁₀ aryl and 4-7-membered heteroaryl containing 1-3 heteroatoms selected from N, O and S;
R₁, R₂, R₃, and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n, and r are selected from the group consisting of: 0, 1 and 2;
W₁ and W₂ are independently selected from the group consisting of: none, C and N; and W₁ and W₂ are not simultaneously N;
ring B is selected from the group consisting of: C₆₋₁₀ aryl, 4-7-membered heteroaryl containing 1-3 heteroatoms selected from N, O and S, saturated or unsaturated C₃₋₁₀ cyclic hydrocarbon group, 3-10-membered heterocyclyl containing 1-3 heteroatoms selected from N, O and S, 3-10-membered bridged heterocyclyl containing 1-3 heteroatoms selected from N, O and S;
VisN;
V' is selected from the group consisting of: -(CH₂)ₚ-, -NH- and -CH₂-NH-;
p is selected from the group consisting of 0 and 1;
X and Y are CH;
R₅ and R₆ are independently selected from the group consisting of: halogen and C₁₋₆alkyl; and the above alkyl is optionally substituted by one or more substituents selected from halogen;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

In another preferred embodiment, 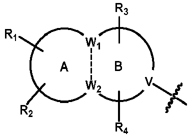 in the formula I is selected from the group consisting of: 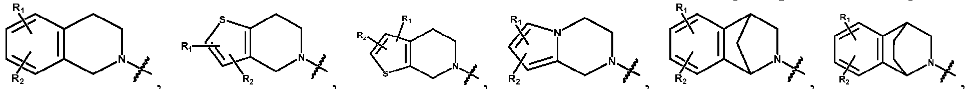 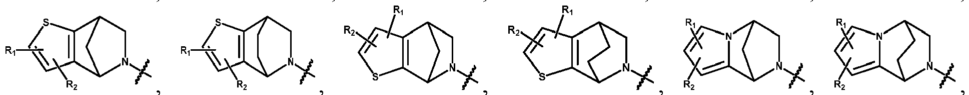 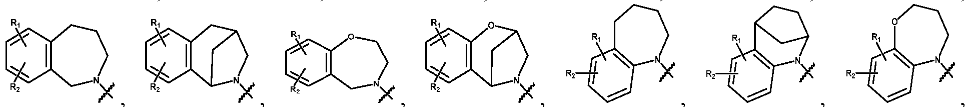 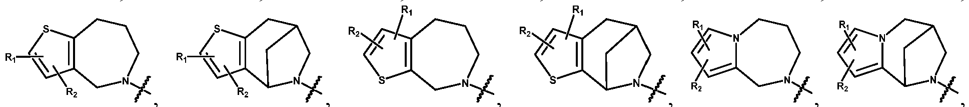 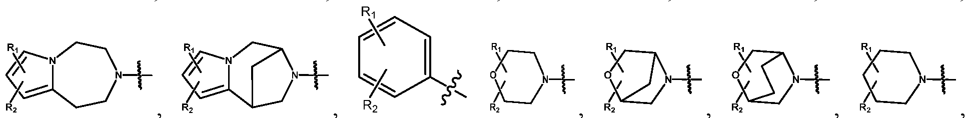 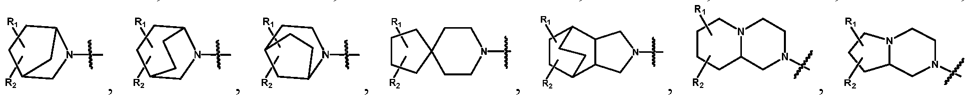 and 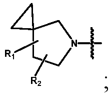
R₁, R₂, R₃ and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n and r are independently selected from the group consisting of: 0, 1 and 2.
In another preferred embodiment,
R₇ is selected from the group consisting of: C₃₋₆ cycloalkyl and C₅₋₈ bridged cyclic group.

In another preferred embodiment, R₇ is selected from the group consisting of: C₃₋₆ cycloalkyl and C₅₋₈ bridged cyclic group, and the above cycloalkyl and bridged cyclic group are optionally substituted by one or more substituents selected from the group consisting of: hydrogen, halogen, cyano, C₁₋₆alkyl and C₁₋₆haloalkyl.

In another preferred embodiment, 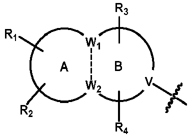 in the formula I is selected from the group consisting of: 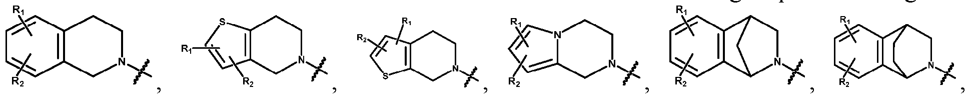 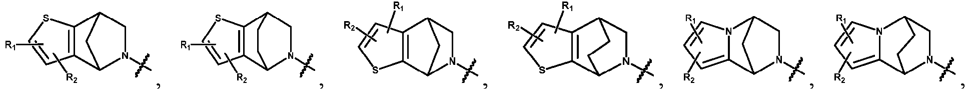 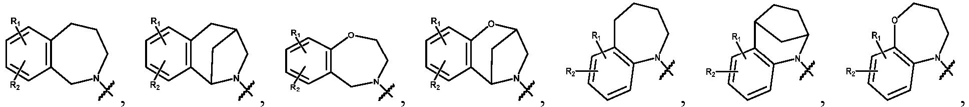 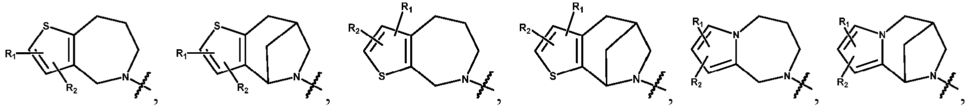 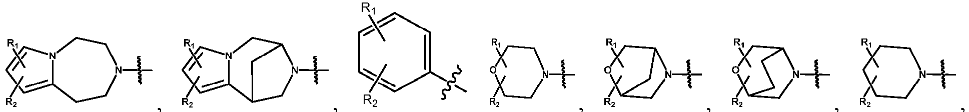 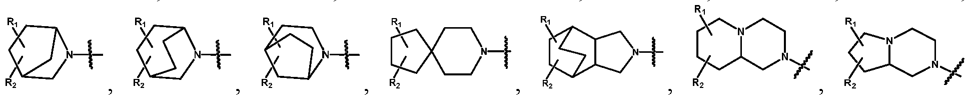 and 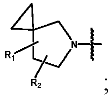
R₁, R₂, R₃ and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n and r are independently selected from the group consisting of: 0, 1 and 2;
V' is selected from the group consisting of: -(CH₂)ₚ-, -NH-, -CH₂-NH- and 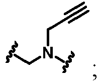
p is selected from the group consisting of 0 and 1;
X and Y are independently selected from the group consisting of: N and CH;
R₅ and R₆ are independently selected from the group consisting of: hydrogen, halogen, amino, C₁₋₆alkyl and C₁₋₆alkoxy;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

In another preferred embodiment,
R₁, R₂, R₃ and R₄ are independently selected from the substituted or unsubstituted group consisting of: hydrogen, halogen, and C₁₋₆alkyl;
n is selected from the group consisting of: 0, 1 and 2;
V' is selected from the group consisting of: -(CH₂)ₚ-, -NH- and -CH₂-NH-;
p is selected from the group consisting of 0 and 1;
X and Y are CH;
R₅ and R₆ are independently selected from the group consisting of: halogen and C₁₋₆alkyl;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

In another preferred embodiment, the compound is selected from the group consisting of:

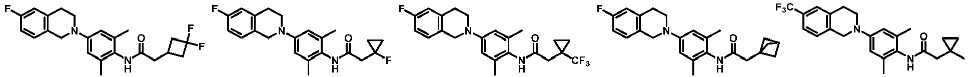

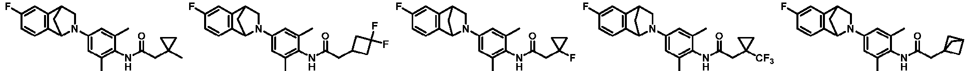

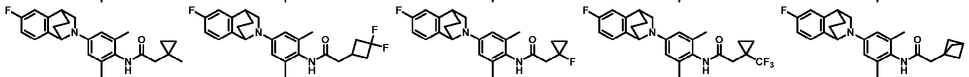

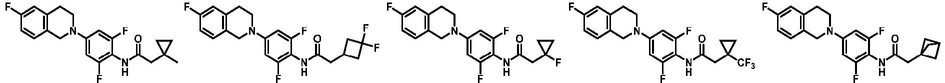

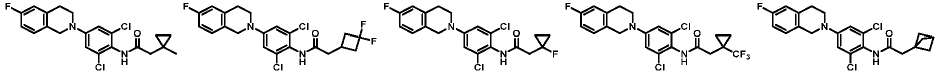

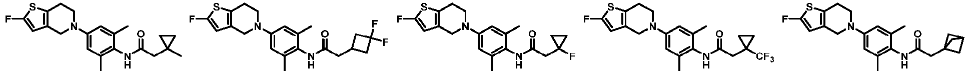

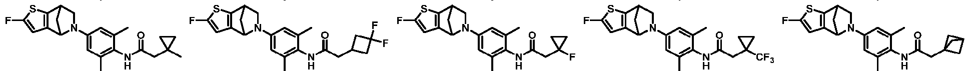

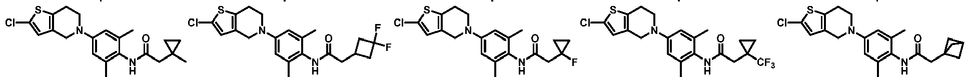

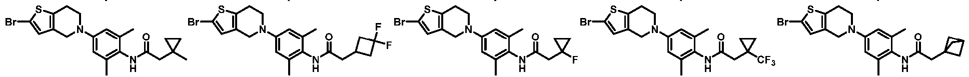

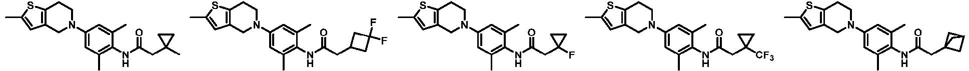

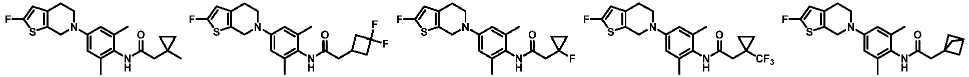

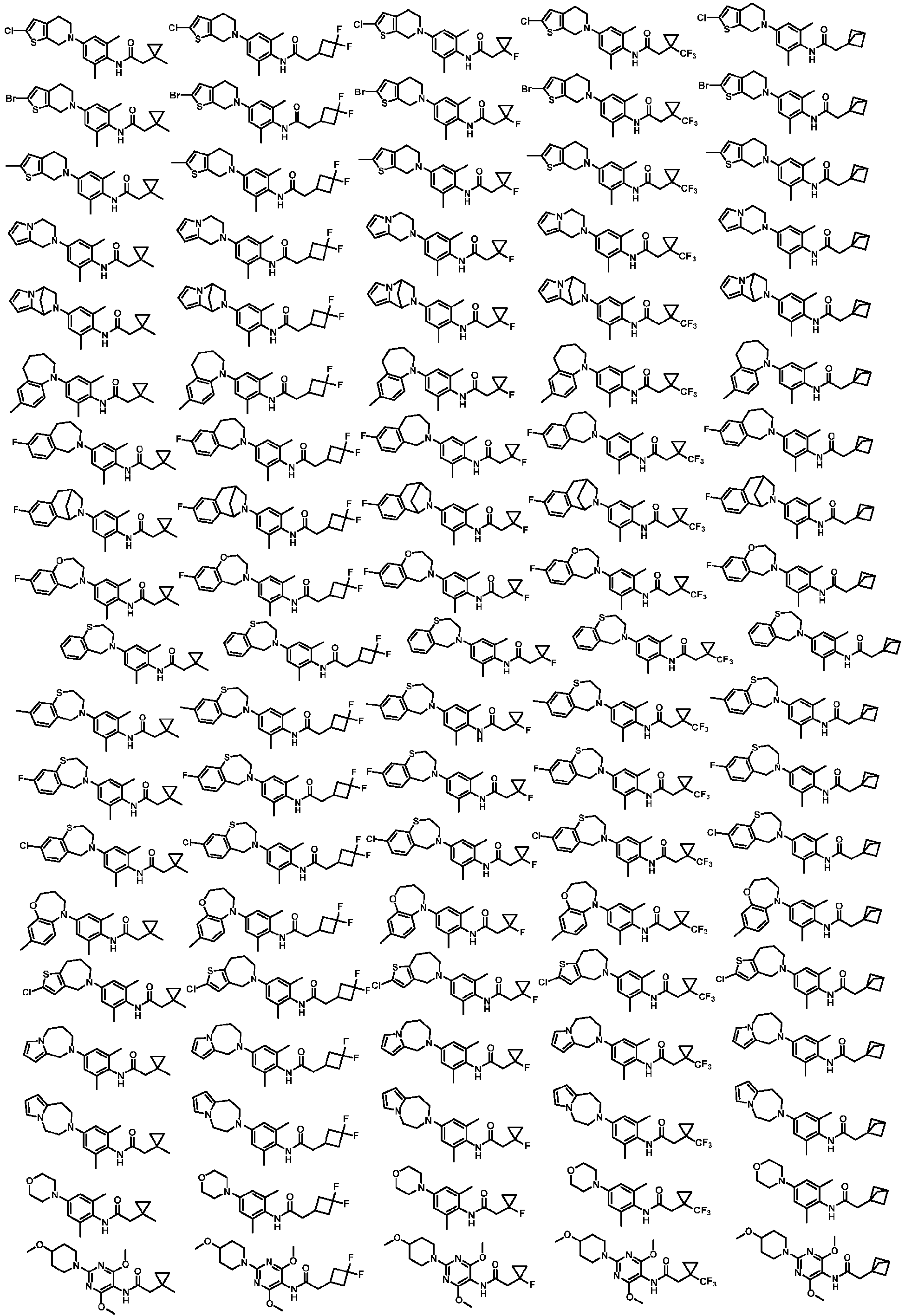

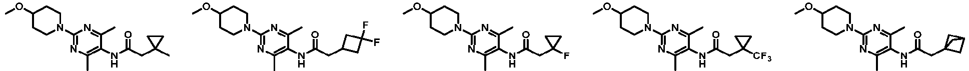

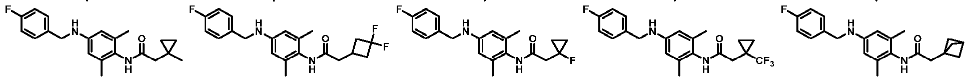

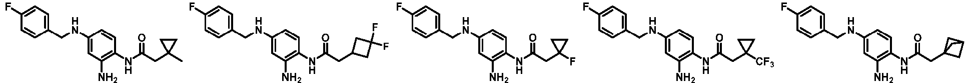

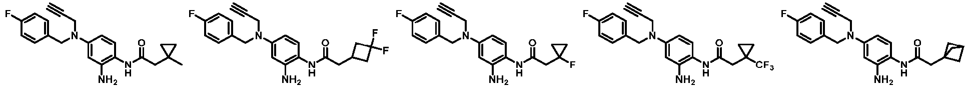

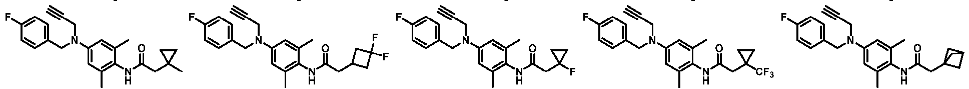

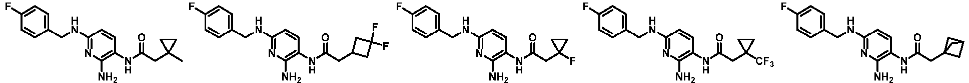

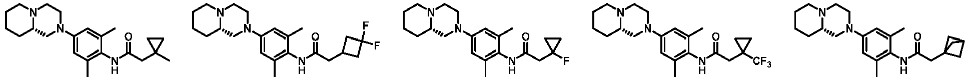

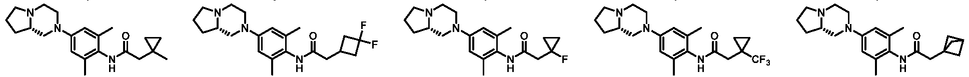

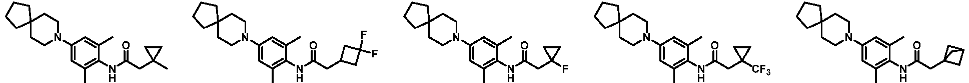

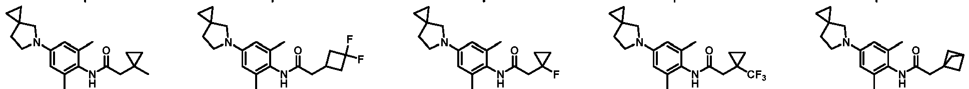

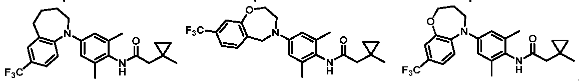

In the second aspect of the present invention, provided is a pharmaceutical composition comprising one or more pharmaceutically acceptable carriers and a therapeutically effective amount of one or more of the compounds described in the first aspect of the present invention or a pharmaceutically acceptable salt thereof.

In the third aspect of the present invention, provided is a use of the compound described in the first aspect of the present invention, or a pharmaceutically acceptable salt thereof in the preparation of a drug for the prevention and/or treatment of a disease that is sensitive to potassium ion channels.

In another preferred embodiment, the disease sensitive to potassium ion channels is a central nervous system disease.

It should be understood that within the scope of the present invention, the above-mentioned technical features of the present invention and the technical features specifically described in the following (such as embodiments) can be combined with each other to form a new or preferred technical solution. Limited to space, it will not be repeated here.

### Detailed Description of the Invention

After long-term and in-depth research, the inventor unexpectedly prepared a compound shown in formula I with excellent potassium channel opening activity, pharmacokinetics (such as brain blood ratio performance), in vivo efficacy and safety, and novel structure through structural optimization. On this basis, the present invention is completed.

### Terms

In the present invention, unless otherwise specified, the terms used herein have the ordinary meanings known to those skilled in the art.

In the present invention, the term "halogen" refers to F, Cl, Br or I.

In the present invention, the term "C₁₋₆ alkyl" refers to a straight or branched alkyl comprising 1-6 carbon atoms, such as methyl, ethyl, propyl, isopropyl, butyl, isobutyl, tert-butyl, neopentyl, tert-pentyl, and the like.

In the present invention, the term "C₂₋₆ alkenyl" refers to a straight or branched alkenyl containing a double bond and 2-6 carbon atoms, and non-limitingly includes vinyl, propenyl, butenyl, isobutenyl, pentenyl, hexenyl, and the like.

In the present invention, the term "C₂₋₆ alkynyl" refers to a straight or branched alkynyl containing a triple bond and 2-6 carbon atoms, and non-limitingly includes ethynyl, propynyl, butynyl, isobutynyl, pentynyl, hexynyl and the like.

In the present invention, the term "C₃₋₆ cyclic hydrocarbon group" includes groups selected from the group consisting of: C₃₋₆ cycloalkyl, C₃₋₆ cycloalkenyl, and C₃₋₆ cycloalkynyl.

The term "C₃₋₆ cycloalkyl" in the present invention refers to a cyclic alkyl having 3-6 carbon atoms on the ring, and non-limitingly includes cyclopropyl, cyclobutyl, cyclopentyl, cyclohexyl, and the like.

In the present invention, the term "C₅₋₈ bridged cyclic group" refers to a cycloalkyl having a bridge bond and 5-8 carbon atoms, and non-limitingly includes 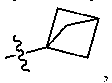 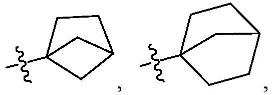 and the like.

In the present invention, the term "C₁₋₆ alkoxy" refers to a straight or branched alkoxy with 1 to 6 carbon atoms, including without limitation methoxy, ethoxy, propoxy, isopropoxy, butoxy, and the like. Preferably refers to C₁₋₄ alkoxy.

In the present invention, the term "heterocyclyl" refers to a 4-8-membered heterocyclyl containing 1, 2, or 3 heteroatoms selected from N, O and S, including, but not limited to, the following groups:

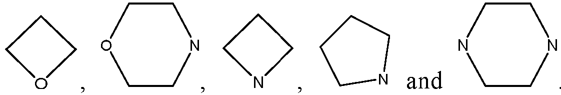

In the present invention, the term "aromatic ring" or "aryl" has the same meaning, preferably "C₆₋₁₀ aryl". The term "C₆₋₁₀ aryl" refers to an aromatic ring group having 6-10 carbon atoms without heteroatoms on the ring, such as phenyl, naphthyl and the like.

In the present invention, the term "aromatic heterocycle" or "heteroaryl" has the same meaning and refers to a heteroaromatic group containing one to more heteroatoms. For example, "C3-C10 heteroaryl" refers to an aromatic heterocyclic ring containing 1 to 4 heteroatoms selected from oxygen, sulfur, and nitrogen and 3 to 10 carbon atoms. Non-limiting examples include: furanyl, thienyl, pyridinyl, pyrazolyl, pyrrolyl, N-alkylpyrrolyl, pyrimidinyl, pyrazinyl, imidazolyl, tetrazolyl and the like. Said heteroaryl ring may be fused to an aryl, heterocyclyl or cycloalkyl ring, wherein the ring attached to the parent structure is a heteroaryl ring. Heteroaryl can be optionally substituted or unsubstituted.

In the present invention, the term "C₆₋₁₂ arylalkyl" refers to an aryl-substituted alkyl having a total of 6-10 carbon atoms without heteroatoms.

In the present invention, the term "halogenated" means being substituted by deuterium.

In the present invention, the term "deuterated" means being substituted by deuterium.

In the present invention, the term "substituted" refers to one or more hydrogen atoms on a particular group are substituted by a particular substituent. The particular substituent is a substituent described above, or a substituent appearing in the Examples. Unless specifically stated, a particular substituted group may have a substituent selected from a particular group at any substitutable site of the group, and the substituents may be the same or different at various positions. It should be understood by those skilled in the art that the combinations of substituents contemplated by the present invention are those that are stable or chemically realizable. The substituents are, for example (but not limited to): halogen, hydroxyl, carboxyl (-COOH), C₁-C₆ alkyl, C₂-C₆ alkenyl, C₂-C₆ alkynyl, C₃-C₈ cycloalkyl, 3- to 12-membered heterocyclyl, aryl, heteroaryl, C₁-C₈ aldehyde, C₂-C₁₀ acyl, C₂-C₁₀ ester group, amino, C₁-C₆ alkoxy, C₁-C₁₀ sulfonyl, etc.

In the present invention, the term 1-6 refers to 1, 2, 3, 4, 5 or 6. Other similar terms have similar meanings independently. The term "more" means 2-6, e.g. 2, 3, 4, 5 or 6.

It should be understood that when a group is present in several different positions of a compound, its definition in each position is independent of each other and may be the same or different. That is, the term "selected from the group consisting of:" has the same meaning as the term "each independently selected from the group consisting of:".

### Compound

The present invention provides a compound of formula I, or a pharmaceutically acceptable salt thereof, 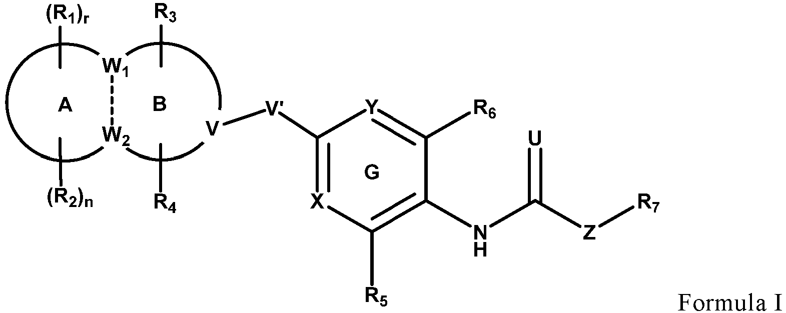 wherein, each group is as defined above.

In another preferred embodiment, any one of ring A, ring B, W₁, W₂, r, n, R₁, R₂, R₃, R₄, R₅, R₆, R₇, V, V', X, Y, U, and Z, respectively, is independently the corresponding group in the specific compound.

In another preferred embodiment, the compound is preferably the compound prepared in each example.

As used herein, the term "pharmaceutically acceptable salt" refers to a salt of a compound of the invention formed with an acid or base suitable for use as a drug. Pharmaceutically acceptable salts include inorganic and organic salts. A preferred class of salts is those formed by the compounds of the present invention with acids. Acids suitable for the formation of salts include, but are not limited to: inorganic acids such as hydrochloric, hydrobromic, hydrofluoric, sulphuric, nitric, and phosphoric acids, etc.; organic acids such as formic, acetic, trifluoroacetic, propionic, oxalic, malonic, succinic, fumaric, maleic, lactic, malic, tartaric, citric, picric, benzoic, methanesulphonic, ethanesulfonic, p-toluenesulphonic, benzenesulphonic, naphthalenesulphonic, etc.; and amino acids such as proline, phenylalanine, aspartic acid, glutamic acid, etc..

Another preferred class of salts are salts formed by the compounds of the present invention with a base, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), such as methylamine salts, ethylamine salts, propylamine salts, dimethylamine salts, trimethylamine salts, diethylamine salts, triethylamine salts, tert-butylamine salts, ethylenediamine salts, hydroxyethylamine salts, dihydroxyethylamine salts, trihydroxyethylamine salts, and amine salts formed from morpholine, piperazine, and lysine, respectively.

It should be understood that the compound of the present invention can be prepared by the method as described in the following examples, and can also be conveniently made by optionally combining various synthetic methods described in this specification or known in the art, and such combinations can be readily carried out by those skilled in the field to which the present invention belongs.

### Pharmaceutical composition and mode of application

The pharmaceutical compositions of the present invention comprise a compound of the present invention or a pharmaceutically acceptable salt thereof within a safe and effective amount of range, and a pharmaceutically acceptable excipient or carrier. The "safe and effective amount" refers to the amount of compound that is sufficient to significantly improve the condition without causing serious side effects. Typically, the pharmaceutical composition contains 1-2000mg compound of the present invention /dose, and more preferably, 5-1000mg compound of the present invention/dose. Preferably, the "one dose" is a capsule or tablet.

"Pharmaceutically acceptable carrier" refers to one or more compatible solid or liquid fillers or gel substances, which are suitable for human use and must have sufficient purity and low toxicity. "Compatibility" herein refers to the ability of components of a composition to blend with each other and with the compounds of the invention without significantly reducing the efficacy of the compounds. Some examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as Tween^{®}), wetting agents (such as sodium dodecyl sulfate), colorants, flavoring agents, stabilizers, antioxidants, preservatives, non-pyrogen raw water, etc.

The pharmaceutical composition is in the form of injection, wafer, tablet, pill, powders or granules.

The mode of administration of the compounds or pharmaceutical compositions of the present invention are not particularly limited, and representative mode of administration include, but are not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, Mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or cassava starch, alginic acid, some complex silicates, and sodium carbonate; (e) slow solvent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets and pills, dosage forms may also contain buffers.

Solid dosage forms such as tablets, sugar pills, capsules and granules may be prepared using coatings and shell materials such as casings and other materials well known in the art. They may comprise an opacifying agent, and the active compound or compounds in such composition may be released in a delayed manner in a part of the digestive tract. Examples of embedding components that can be employed are polymeric substances and wax substances. If necessary, the active compound may also form a microcapsule with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and spices.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compound of the invention for topical administration include ointments, powders, patches, propellants and inhalants. The active ingredient is mixed under sterile conditions with a physiologically acceptable carrier and any preservatives, buffers, or propellants as may be required.

The compound of the present invention can be administered alone or in combination with other pharmaceutically acceptable compounds.

The treatment method of the present invention can be administered alone or in combination with other treatment methods or drugs.

When using the pharmaceutical composition, a safe and effective amount of the compound of the present invention is administered to mammals (such as humans) in need of treatment, wherein the dosage at the time of administration is the pharmaceutically considered effective dose, which is typically 1- 2000 mg per day, more preferably 5-1000mg per day for a 60 kg body weight human. Of course, the specific dosage should also consider the route of administration, the patient's health condition and other factors, which are all within the skill range of skilled doctors.

### Compared with the prior art, the main advantages of the present invention include:

(1) The compound exhibits superior pharmacokinetic properties, such as better brain blood ratio, half-life period, exposure level, metabolic stability, etc;
(2) The compound has better potassium ion channel opening activity, better potassium ion channel activation rate, better ion channel selectivity, better in vivo efficacy, and better safety;
(3) The compound is expected to be used in the treatment and/or prevention of diseases and conditions affected by the activity of potassium ion channels.

The present invention is further described below in conjunction with specific embodiments. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

Unless otherwise defined, all professional and scientific terms used herein have the same meanings as commonly understood by those skilled in the art. In addition, any methods and materials similar or equivalent to those described can be applied to the method of the present invention. The preferred embodiments and materials described herein are for exemplary purposes only.

The experimental materials and reagents used in the following examples are from commercially available sources unless otherwise specified.

### Example 1 Preparation of Compound 3001

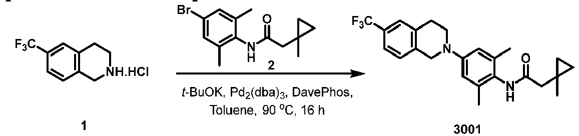

### Step 1, compound 3001

Compound 1 (100 mg, 0.420 mmol, 1.0 eq) was dissolved in toluene (6 mL), and compound 2 (149 mg, 0.50 mmol, 1.2 eq), potassium tert-butoxide (142 mg, 1.26 mmol, 3.0 eq), Dave-Phos (17 mg, 0.042 mmol, 0.1 eq) and Pd₂(dba)₃ (19 mg, 0.021 mmol, 0.05 eq) were added sequentially. The reaction was heated to 90 °C under nitrogen atmosphere and stirred for 16 hours. After cooling to 25 °C, the reaction solution was diluted with ethyl acetate (30 mL), then washed with water and saturated sodium chloride solution sequentially, dried over anhydrous sodium sulfate and concentrated; then the obtained residue was purified by pre-HPLC (0.1% formic acid/acetonitrile/water) to give compound **3001** (65.4 mg, 37%).
LCMS: [M+H] ⁺ = 417.2
¹H NMR (400 MHz DMSO-*d*₆) δ 8.81 (s, 1H), 7.54-7.52 (m, 2H), 7.47-7.45 (m, 1H), 6.74 (s, 2H), 4.43 (s, 2H), 3.52 (t, J = 5.8 Hz, 2H), 2.99 (t, J = 5.8 Hz, 2H), 2.17 (s, 2H), 2.11 (s, 6H), 1.14 (s, 3H), 0.53 (q, J = 4.1 Hz, 2H), 0.31 (q, J = 4.1 Hz, 2H).

### Example 2 Preparation of Compound 3002

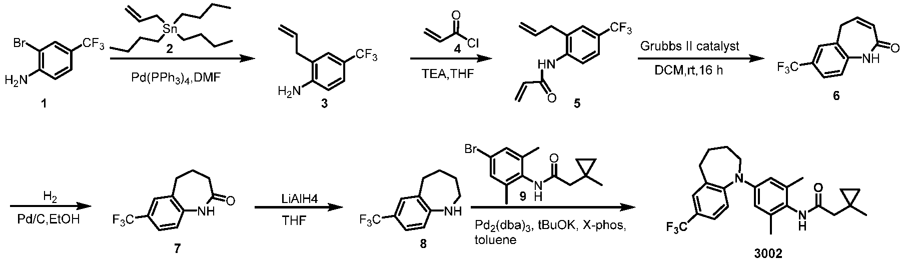

### Step 1, compound 3

Compound **1** (5.0 g, 20.0 mmol, 1.0 eq) was dissolved in N,N-dimethylformamide (50 mL), and compound **2** (6.65 g, 20.0 mmol, 1.0 eq) and Pd(PPh₃)₄ (700 mg, 0.6 mmol, 0.03 eq) were added sequentially, and heated up to 80 °C and stirred for 16 hours. The reaction solution was diluted with ethyl acetate (50 mL), washed with saturated sodium chloride solution, dried over anhydrous sodium sulfate and concentrated; then the residue was purified by silica gel column chromatography (petroleum ether/ethyl acetate=10/1) to give compound **3** (3.5 g, 87%) as a white solid.
LCMS: [M+H] ⁺ = 202.2

### Step 2, compound 5

Compound **3** (1.5 g, 7.46 mmol, 1.0 eq) was dissolved in tetrahydrofuran (20 mL) and then cooled to 0 °C. Compound **4** (810 mg, 8.95 mmol, 1.2 eq) and triethylamine (2.3 g, 22.4 mmol, 3.0 eq) were added. The reaction solution was stirred at 25 °C under nitrogen protection for 1 h, then diluted with ethyl acetate (50 mL) and washed with saturated saline; the organic phase was dried with anhydrous sodium sulfate and concentrated, and then the residue was purified by silica gel column chromatography (petroleum ether/ethyl acetate=10/1) to give compound 5 (1.5 g, 79%) as a white solid.
LCMS: [M+H] ⁺ =256.1

### Step 3, compound 6

Compound **5** (1.5 g, 5.9 mmol, 1.0 eq) was dissolved in dichloromethane (1 mL), and Grubbs II catalyst (300 mg, 0.353 mmol, 0.06 eq) was added under nitrogen protection. The reaction solution was stirred at 25 °C for 16 h under nitrogen protection, then diluted with ethyl acetate (50 mL), and washed with saturated saline, the organic phase was dried over anhydrous sodium sulfate and concentrated, and then the residue was purified by silica gel column chromatography (petroleum ether/ethyl acetate=2/1) to give compound **6** (900 mg, 67%) as a white solid.
LCMS: [M+H] ⁺ =228.1

### Step 4, compound 7

Compound **6** (900 mg, 3.961 mmol, 1.0 eq) was dissolved in ethanol (20 mL), Pd/C (10%, 90 mg) was added, and the reaction solution was stirred overnight at 25 °C under hydrogen atmosphere at 1 atmosphere. The filtrate obtained from filtration was concentrated to give compound **7** (900 mg, 99%) as a colorless oil.
LCMS: [M+H] ⁺ =230.1

### Step 5, compound 8

Compound **7** (400 mg, 1.75 mmol, 1.0 eq) was dissolved in tetrahydrofuran (30 mL) and cooled to 0 °C; a solution of borane in tetrahydrofuran (1 M, 35 mL, 35 mmol, 20.0 eq) was added, then heated up to 50 °C and stirred for 2 hours. After cooling to 0 °C, methanol (30 mL) was added and stirred for 0.5 h. The reaction solution was concentrated to give compound **8** (400 mg, 99%) as a colorless oil.
LCMS: [M+H] ⁺ = 216.2

### Step 6, compound 3002

Compound **8** (100 mg, 0.47 mmol, 1.0 eq) was dissolved in toluene (5 mL), and compound **9** (139 mg, 0.47 mmol, 1.0 eq), potassium tert-butoxide (158 mg, 1.41 mmol, 3.0 eq), X-Phos (45 mg, 0.094 mmol, 0.2 eq) and Pd₂(dba)₃ (43 mg, 0.047 mmol, 0.1 eq) were added, then heated up to 99 °C and stirred for 16 hours. After cooling to 25 °C, the reaction solution was filtered and concentrated, and then the obtained residue was purified by pre-HPLC (0.1% formic acid/acetonitrile/water) to give compound **3002** (41.6 mg, 21%).
LCMS: [M+H] ⁺ = 431.2
¹H NMR (400 MHz, DMSO-*d*₆) δ 8.77 (s, 1H), 7.69 (s, 1H), 7.53 (d, *J =* 8.0 Hz, 1H), 7.22 (d, *J =* 8.0 Hz, 1H), 6.42 (s, 2H), 3.63 (s, 2H), 2.76 (s, 2H), 2.17 (s, 2H), 2.03 (s, 6H), 1.73 (s, 2H), 1.65 (s, 2H), 1.13 (s, 3H), 0.55-0.51 (m, 2H), 0.32-0.28 (m, 2H).

### Example 3 Preparation of Compound 3003

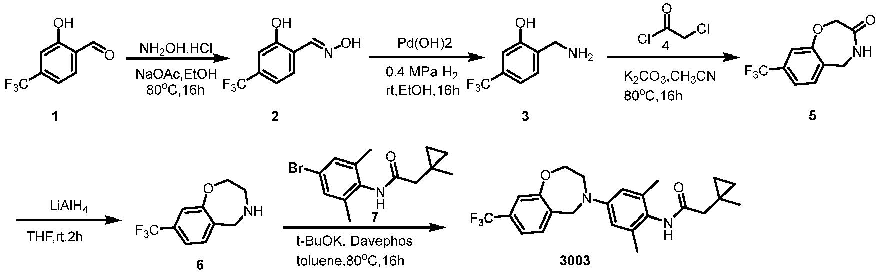

### Step 1, compound 2

Compound **1** (1.0 g, 5.26 mmol, 1.0 eq) was dissolved in ethanol (20 mL), hydroxylamine hydrochloride (1.24 g, 17.9 mmol, 3.4 eq) and sodium acetate (1.73 g, 21.04 mmol, 4.0 eq) were added, and the reaction solution was heated up to 80 °C and stirred for 16 hours. The reaction solution was cooled to room temperature and concentrated, the residue was diluted with ethyl acetate and filtered, and the filtrate was washed with saturated saline and dried over anhydrous sodium sulfate, then concentrated to give compound **2** (1.2 g, 100%).
LCMS: [M+H] ⁺ = 206.2

### Step 2, compound 3

Compound **2** (1.2 g, 5.26 mmol) was dissolved in ethanol (30 mL), concentrated hydrochloric acid (12 M, 3 mL) and palladium hydroxide (0.4 g) were added, and the reaction solution was reacted at room temperature for 16 hours under hydrogen atmosphere at 0.4 MPa. The pH of the reaction solution was adjusted to 8 with saturated aqueous sodium bicarbonate solution and the reaction solution was filtered. The filtrate was concentrated, the resulting residue was diluted with ethyl acetate and washed with saturated sodium chloride solution, and the organic phase was dried over anhydrous sodium sulfate and then concentrated to give compound **3** (1.0 g, 99 %).
LCMS: [M+H] ⁺ = 192.1

### Step 3, compound 5

Compound **3** (1.0 g, 5.23 mmol, l.0 eq) was dissolved in acetonitrile (50 mL), potassium carbonate (2.89 g, 20.93 mmol, 4.0 eq), tetrabutylammonium bromide (0.169 g, 0.52 mmol, 0.1 eq), and chloroacetyl chloride (0.886 g, 0.7.85 mmol, 1.5 eq) were added sequentially, and heated up to 80 °C and stirred for 16 hours. The reaction solution was cooled to room temperature and filtered, the filtrate was concentrated, and then the obtained residue was purified by silica gel column chromatography (petroleum ether/ethyl acetate=1/1) to give compound **5** (0.8 g, 66 %).
LCMS: [M+H] ⁺ = 232.1

### Step 4, compound 6

Compound **5** (800 mg, 3.46 mmo, 1.0 eq) was dissolved in tetrahydrofuran (30 mL) and cooled to 0 °C, a solution of lithium tetrahydroaluminate in tetrahydrofuran (1 M, 13 mL, 13 mmol, 3.7 eq) was added, and heated up to 25 °C and stirred for 2 hours. The reaction solution was diluted with tetrahydrofuran (200 mL), added with sodium sulfate decahydrate (4.0 g) and stirred for 2 hours. After filtration, the filtrate was concentrated to give compound 6 (600 mg, 80%).
LCMS: [M+H] ⁺ = 218.**1**

### Step 5, compound 3003

Compound **6** (162 mg, 0.74 mmol, 1.0 eq) was dissolved in toluene (20 mL), compound **7** (221 mg, 0.74 mmol, 1.0 eq), Pd₂(dba)₃ (68 mg, 0.07 mmol, 0.1 eq), Davephos (59 mg, 0.15 mmol, 0.2 eq) and Potassium tert-butoxide (251 mg, 2.24 mmol, 3.0 eq) were added sequentially, then heated to 80 °C and stirred for 16 hours. The reaction solution was cooled to room temperature and filtered, the filtrate was concentrated, and then the obtained residue was purified by pre-HPLC (0.1% formic acid/acetonitrile/water) to give compound **3003** (50.9 mg, 16%).
LCMS: [M+H] ⁺ = 433.2
¹H NMR (400 MHz, DMSO-*d*₆) δ 8.67 (s, 1H), 7.75 (d, *J =* 7.6 Hz, 1H), 7.37 (d, *J* = 8.0 Hz, 1H), 7.22 (s, 1H), 6.58 (s, 2H), 4.69 (s, 2H), 4.24 - 4.10 (m, 2H), 3.94 - 3.80 (m, 2H), 2.13 (s, 2H), 2.03 (s, 6H), 1.11 (s, 3H), 0.52-0.49 (m, 2H), 0.29-0.26 (m, 2H).

### Example 4 Preparation of Compound 3004

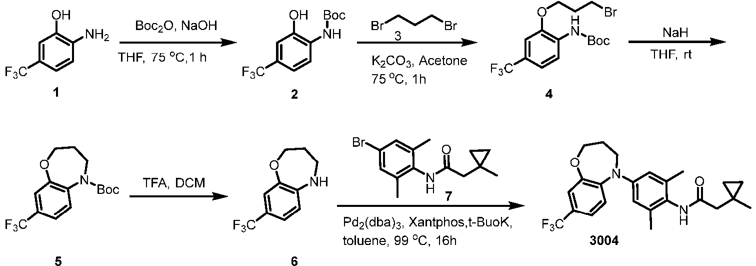

### Step 1, compound 2

Compound **1** (330 mg, 1.86 mmol, 1.0 eq) was dissolved in anhydrous THF (10 mL), BoczO (406 mg, 1.86 mmol, 1.0 eq) and NaOH (223 mg, 5.59 mmol, 3.0 eq) were added sequentially, and the reaction solution was heated up to 75 °C under nitrogen protection and stirred for 1 h. The reaction solution was poured into ice water and extracted with ethyl acetate (3 × 50 mL); the organic phase was washed with water and saturated sodium chloride solution sequentially, dried over anhydrous sodium sulfate and concentrated, and then the obtained residue was purified by silica gel column chromatography (petroleum ether/ethyl acetate = 10/1) to give Compound **2** (330 mg, 64 %) as a white solid.
LCMS: [M-t-Bu+H] ⁺ = 222.1

### Step 2, compound 4

Compound **2** (250 mg, 0.90 mmol, 1.0 eq) was dissolved in acetone (5 mL), 1,3-dibromopropane (637 mg, 3.16 mmol, 3.5 eq) and potassium carbonate (1.0 g, 7.21 mmol, 8.0 eq) were added, and the reaction solution was heated up to 75 °C under nitrogen protection and stirred for 1 h. After cooling to room temperature, the reaction solution was concentrated and diluted with ethyl acetate (50 mL) and water (50 mL), the separated aqueous phase was extracted with ethyl acetate (3 × 50 mL) and the organic phases were combined. The organic phase was washed with water and saturated sodium chloride solution sequentially, dried over anhydrous sodium sulfate and concentrated, and then the obtained residue was purified by silica gel column chromatography (petroleum ether/ethyl acetate = 6/1) to give compound **4** (250 mg, 70 %) as a yellow oily substance.
LCMS: [M-t-Bu+H] ⁺ = 342.0

### Step 3, compound 5

Compound **4** (300 mg, 0.75 mmol, 1.0 eq) was dissolved in anhydrous THF (20 mL), NaH (60%, 180 mg, 4.52 mmol, 6.0 eq) was added, and the reaction solution was stirred at 25 °C for 1 hour. The reaction solution was quenched by pouring into ice water and extracted with ethyl acetate (3 × 50 mL), the combined organic phases were washed with water and saturated sodium chloride solution sequentially, dried over anhydrous sodium sulfate and concentrated, and the residue was purified by silica gel column chromatography (petroleum ether/ethyl acetate = 50/1) to give compound **5** (160 mg, 66 %).
LCMS: [M-t-Bu+H] ⁺ = 262.0

### Step 4, compound 6

Compound **5** (160 mg, 0.50 mmol, 1.0 eq) was dissolved in dichloromethane (5 mL), trifluoroacetic acid (3 mL) was added, and stirred at 25 °C for 1 hour. The reaction solution was adjusted to pH 7-8 with saturated aqueous sodium carbonate solution and extracted with ethyl acetate (3×50 mL), the combined organic phases were washed with water and saturated aqueous sodium chloride sequentially, dried over anhydrous sodium sulfate and then concentrated, and the residue was purified by silica gel column chromatography (petroleum ether/ethyl acetate= 3/1) to give compound **6** (80 mg, 73 %) as a white solid.
LCMS: [M+H] ⁺ = 218.1

### Step 5, compound 3004

Compound **6** (50 mg, 0.23 mmol, 1.0 eq) was dissolved in toluene (10 mL), compound **7** (75 mg, 0.25 mmol 1.1 eq), t-BuoK (77 mg, 0.69 mmol, 3.0 eq), Xantphos (26 mg, 0.04 mmol, 0.2 eq) and Pd₂(dba)₃ (21 mg, 0.02 mmol, 0.1 eq) were added, and heated to 99 °C under nitrogen protection and stirred overnight. After cooling to room temperature, the reaction solution was diluted with ethyl acetate (10 mL), then washed with water and saturated sodium chloride solution sequentially, dried over anhydrous sodium sulfate and concentrated, and the residue was purified by Pre-HPLC (0.1% formic acid/acetonitrile/water) to give compound **3004** (15.2 mg, 15 %) as a white solid.
LCMS: [M+H] ⁺ = 433.2
¹H NMR (400 MHz, DMSO-*d*₆) δ 8.85 (s, 1H), 7.24-7.21 (m, 2H), 7.11-7.09 (m, 1H), 6.76 (s, 2H), 4.17-4.14 (m, 2H), 3.91-3.88 (m, 2H), 2.19 (s, 2H), 2.07 (s, 6H), 2.00-1.97 (m, 2H), 1.14 (s, 3H), 0.54-0.53 (m, 2H), 0.33-0.30 (m, 2H).

### Example 5 Preparation of Compound 3005

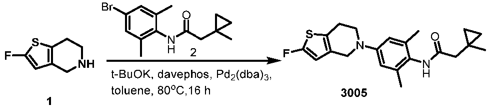

### Step 1, compound 3005

Compound **1** (120 mg, 0.76 mmol, 1.0 eq) was dissolved in toluene (3 mL), compound **2** (271 mg, 0.92 mmol, 1.2 eq), potassium t-butoxide (257 mg, 2.29 mmol, 3.0 eq), Dave-Phos (60 mg, 0.15 mmol, 0.2 eq) and Pd₂(dba)₃ (70 mg, 0.076 mmol, 0.1 eq) were added sequentially, and heated up to 80 °C and stirred for 16 hours. After cooling to room temperature, the reaction solution was filtered, the filtrate was concentrated, and then the obtained residue was purified by pre-HPLC (0.1% formic acid/acetonitrile/water) to give compound **3005** (41.3 mg, 15 %).
LCMS: [M+H] ⁺ = 373.2
¹H NMR (400 MHz, DMSO-*d*₆) δ 8.80 (s, 1H), 6.70 (s, 2H), 6.52 (d, J = 2.4 Hz, 1H), 4.10 (s, 2H), 3.55 (t, J = 5.6 Hz, 2H), 2.76 (s, 2H), 2.17 (s, 2H), 2.09 (s, 6H), 1.14 (s, 3H), 0.55 - 0.52 (m, 2H), 0.32 - 0.29 (m, 2H).

### Example 6 Preparation of Compound 3006

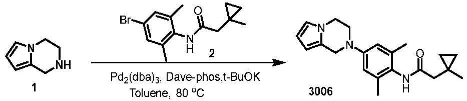

### Step 1, compound 3006

Compound 1 (74 mg, 0.6082 mmol, 1.2 eq) was dissolved in toluene (20 mL), compound 2 (150 mg, 0.5068 mmol, 1.0 eq), Pd₂(dba)₃ (46 mg, 0.05068 mmol, 0.1 eq), Dave-phos (40 mg, 0.1014 mmol, 0.2 eq) and t-BuOK (170 mg, 1.5204 mmol, 3.0 eq) were added, and heated up to 80°C and stirred for 16 hours. After cooling to room temperature, the reaction solution was diluted with ethyl acetate (50 mL), washed with water and saturated sodium chloride solution sequentially, dried over anhydrous sodium sulfate and concentrated, and the residue was purified by silica gel column chromatography (petroleum ether/ethyl acetate=3/1) to give compound **3006** (96 mg, 56%).
LCMS: [M+H] ⁺ = 338.2
¹H NMR (400 MHz, DMSO-*d*₆) δ 8.82 (s, 1H), 6.72 (s, 2H), 6.65 - 6.64 (m, 1H), 6.01 - 6.00 (m, 1H), 5.84 - 5.82 (m, 1H), 4.32 (s, 2H), 4.03 - 4.00 (m, 2H), 3.61 - 3.59 (m, 2H), 2.17 (s, 2H), 2.10 (s, 6H), 1.14 (s, 3H), 0.55 - 0.52 (m, 2H), 0.32 - 0.30 (m, 2H).

### Example 7 Preparation of Compound 3007

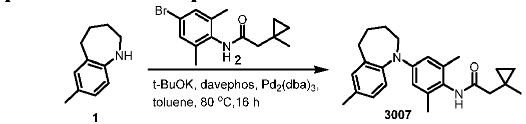

### Step 1, compound 3007

Compound **1** (120 mg, 0.744 mmol, 1.0 eq) was dissolved in toluene (10 mL), compound **2** (264 mg, 0.893 mmol, 1.2 eq), potassium t-butoxide (252 mg, 2.232 mmol, 3.0 eq), Dave-Phos (60 mg, 0.149 mmol, 0.2 eq) and Pd₂(dba)₃ (72 mg, 0.074 mmol, 0.1 eq) were added sequentially, and heated to 80 °C and stirred for 16 hours. After cooling to room temperature, the reaction solution was filtered, and the filtrate was diluted with water and extracted with ethyl acetate (20 mL × 3). The combined organic phases were washed with saturated sodium chloride solution, dried over anhydrous sodium sulfate and concentrated, and the residue was purified by pre-HPLC (0.1% formic acid/acetonitrile/water) to give compound **3007** (53.2 mg, 19%) as a white solid.
LCMS: [M+H] ⁺ = 377.3
¹H NMR (400 MHz, DMSO-*d*₆) δ 8.68 (s, 1H), 7.13 (s, 1H), 7.06 - 7.04 (m, 1H), 6.99-6.97 (m, 1H), 6.22 (s, 2H), 3.56 (s, 2H), 2.55 - 2.52 (m, 2H), 2.30 (s, 3H), 2.15 (s, 2H), 1.99 (s, 6H), 1.78-1.70 (m, 2H), 1.64 - 1.56 (m, 2H), 1.12 (s, 3H), 0.53-0.51 (m, 2H), 0.31 - 0.28 (m, 2H).

### Example 8 Preparation of Compound 3008

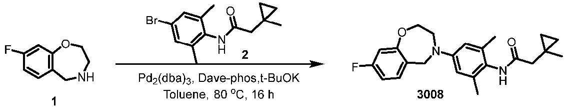

### Step 1, compound 3008

Compound **1** (400 mg, 2.39 mmol, 1.0 eq) was dissolved in toluene (45 mL), compound **2** (779 mg, 2.63 mmol, 1.1 eq), Pd₂(dba)₃ (218 mg, 0.239 mmol, 0.1 eq), Dave-phos (188 mg, 0.478 mmol. 0.2 eq) and t-BuOK (804 mg, 7.17 mmol, 3.0 eq) were added sequentially, and heated up to 80°C and stirred for 16 hours. After cooling to room temperature, the reaction solution was diluted with ethyl acetate (50 mL), washed with water and saturated sodium chloride solution sequentially, dried over anhydrous sodium sulfate and concentrated, and the residue was purified by silica gel column chromatography (petroleum ether / ethyl acetate = 3/1) to give compound **3008** (500 mg, 55%).
LCMS: [M+H] ⁺ = 383.1
¹H NMR (400 MHz, DMSO-*d*₆) δ 8.68 (s, 1H), 7.54-7.50 (m, 1H), 6.86-8.82 (m, 1H), 6.78-6.75 (m, 1H), 6.56 (s, 2H), 4.58 (s, 2H), 4.13 - 4.11 (m, 2H), 3.84 - 3.82 (m, 2H), 2.13 (s, 2H), 2.03 (s, 6H), 1.11 (s, 3H), 0.52-0.49 (m, 2H), 0.30-0.28 (m, 2H).

### Example 9 Preparation of Compound 3009

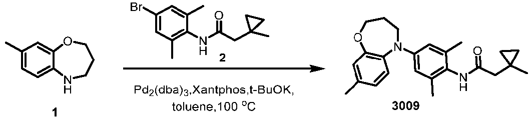

### Step 1, compound 3009

Compound **1** (140 mg, 0.86 mmol, 1.2 eq) was dissolved in toluene, compound **2** (211 mg, 0.72 mmol, 1.0 eq), XantPhos (165 mg, 0.286 mmol, 0.4 eq), potassium tert-butoxide (240 mg, 2.14 mmol, 3.0 eq) and Pd₂(dba)₃ (82 mg, 0.14 mmol 0.2 eq) were added sequentially, and heated to 120 °C and stirred for 4 hours. After cooling to room temperature, the reaction solution was poured into water and extracted with ethyl acetate (50 mL × 2), the combined organic phases were washed with saturated sodium chloride solution, dried over anhydrous sodium sulfate and concentrated, and the residue was purified by silica gel column chromatography (petroleum ether/ethyl acetate=4/1) to give compound **3009** (97.8 mg, 36%) as a white solid.
LCMS: [M+H] ⁺ = 379.3.
¹H NMR (400 MHz, DMSO-d6): δ 8.74 (s, 1H), 6.96-6.94 (m, 1H), 6.83-6.79 (m, 2H), 6.49 (s, 2H), 3.99-3.96 (m, 2H), 3.79-3.76 (m, 2H), 2.25 (s, 3H), 2.16 (s, 2H), 2.02 (s, 6H), 1.99-1.96 (m, 2H), 1.13 (s, 3H), 0.52-0.51 (m, 2H), 0.31-0.29 (m, 2H).

### Example 10 Preparation of Compound 3010

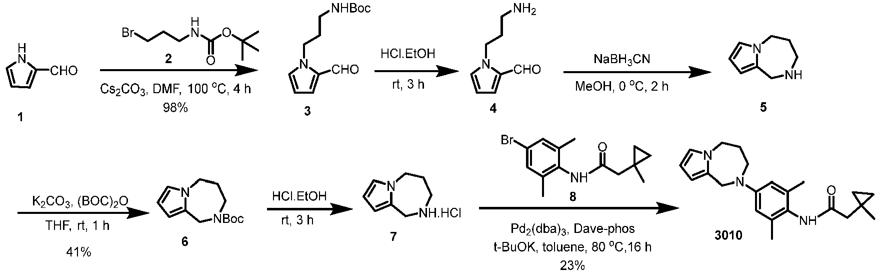

### Step 1, compound 3

To 30 mL of N,N-dimethylformamide was added Compound **1** (2 g, 21 mmol), Compound **2** (6.01 g, 25.2 mmol) and cesium carbonate (10.28 g, 31.5 mmol) sequentially, and the reaction solution was heated up to 100°C for 4 hours. After cooling to room temperature, 100 mL of ethyl acetate and 100 mL of water were added, the organic phase was separated out, and the aqueous phase was extracted with ethyl acetate three times. The combined organic phases were washed with saturated saline, dried over anhydrous sodium sulfate, and concentrated to give compound **3** (6.2 g, 98% yield) as a yellow liquid.
LCMS: [M+Na]⁺ = 275.1

### Step 2, compound 4

To 10 mL of hydrochloric acid-ethanol solution was added Compound **3** (2 g, 7.93 mmol) and stirred at 25°C for 3 hours. 100 mL of ethyl acetate and 100 mL of water were added, the aqueous phase was separated and the organic phase was concentrated to give compound **4** (1.2 g, crude).
LCMS: [M+H]⁺ = 153.1

### Step 3, compound 5

To 20 mL of methanol was added Compound **4** (1.2 g, crude) and NaBH₃CN (2.1 g, 33.6 mmol), and stirred at 0 °C for 2 hours. The reaction solution was diluted with 20 mL of methanol, filtered and dried, and concentrated to give compound **5** (1.1 g, crude, yellow liquid).
LCMS: [M+H]⁺ = 137.2

### Step 4, compound 6

To 20 mL of tetrahydrofuran was added Compound **5** (1.05 g, crude), (Boc)₂O (2.1 g, 9.6 mmol) and potassium carbonate (2.2 g, 15.9 mmol), and stirred for 1 hour at 25°C. 100 mL of ethyl acetate and 100 mL of water were added, the organic phase was separated out, and the aqueous phase was extracted with ethyl acetate three times. The combined organic phases were washed with saturated sodium chloride solution, dried over anhydrous sodium sulfate and concentrated, and the residue was purified by column chromatography (petroleum ether: ethyl acetate=3:1) to give compound 6 (685 mg, 41% yield) as a yellow liquid.
LCMS: [M+H]⁺ = 237.2

### Step 5, compound 7

To 6 mL of hydrochloric acid-ethanol solution was added Compound **6** (300 mg, 1.271 mmol), and stirred at 25°C for 3 hours. The above reaction solution was concentrated to give compound **7** (252 mg, yellow liquid).
LCMS: [M+H]⁺ = 137.2

### Step 6, compound 3010

To 10 mL of toluene was added Compound 7 (252 mg, 1.47 mmol), Compound **8** (250 mg, 0.848 mmol), Pd₂(dba)₃ (78 mg, 0.085 mmol), Dave-phos (68 mg, 0.17 mmol) and potassium tert-butoxide (284 mg, 2.5 mmol), and heated to 80°C under nitrogen protection and stirred for 16 hours. After cooling to room temperature, the above reaction solution was concentrated, and the residue was purified by Pre-HPLC to give compound **3010** (115 mg, 23% yield).
LCMS: [M+H]⁺ = 352.2
¹H NMR (400 MHz, DMSO-*d₆*) *δ* 8.66 (s, 1H), 6.56 - 6.54 (m, 1H), 6.52 (s, 2H), 6.02 (dd, *J =* 3.2, 1.6 Hz, 1H), 5.73 - 5.70 (m, 1H), 4.48 (s, 2H), 4.14 - 4.07 (m, 2H), 3.73 (s, 2H), 2.13 (s, 2H), 2.02 (s, 6H), 1.73 (d, *J =* 4.4 Hz, 2H), 1.11 (s, 3H), 0.51 (q, *J =* 4.4 Hz, 2H), 0.28 (q, *J =* 4.0 Hz, 2H).

### Example 11 Preparation of Compound 3011

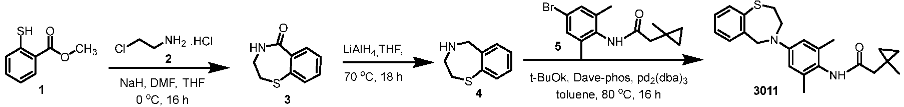

### Step 1, compound 3

To 40 mL of a mixed solvent (N,N-dimethylformamide/tetrahydrofuran = 1/1) was added Compound **1** (4.0 g, 23.8 mmol) and Compound **2** (2.8 g, 24 mmol), and cooled to 0°C and then NaH (60%, 2.9 g, 120 mmol) was added, and heated to 25°C and stirred for 16 hours. The reaction solution was diluted with 200 mL of ice water and extracted three times with ethyl acetate, the combined organic phases were washed with saturated sodium chloride solution and dried over anhydrous sodium sulfate and concentrated. The residue was purified by silica gel column chromatography (petroleum ether/ethyl acetate=4/1) to give compound **3** (1.2 g, 30% yield).
LCMS: [M+H]⁺ = 180.1

### Step 2, compound 4

Compound **3** (1.2 g, 6.9 mmol) was dissolved in 20 mL of tetrahydrofuran, a solution of LiAlH₄ (1M, 10.5 mL, 10.5 mmol) in tetrahydrofuran was added, and heated up to 70°C and stirred for 18 hours. After cooling to room temperature, the reaction was quenched by adding sodium sulfate decahydrate and filtrated, and then the obtained filtrate was concentrated to give compound **4** (1.1 g, 95% yield) as a yellow oily substance.
LCMS: [M+H]⁺ = 166.1

### Step 3, compound 3011

To 10 mL of toluene was added Compound **4** (308 mg, 1.87 mmol), Compound **5** (500 mg, 1.7 mmol) and potassium tert-butoxide (570 mg, 5.1 mmol); Dave-phos (134 mg, 0.34 mmol) and pd₂(dba)₃ (156 mg, 0.17 mmol) were added sequentially, and the reaction solution was heated up to 80°C under nitrogen protection and stirred for 16 hours. After cooling to room temperature, the reaction solution was filtered, the filtrate was concentrated, and then the obtained residue was purified by silica gel column chromatography (petroleum ether/ethyl acetate=3/1) to give compound **3011** (475 mg, 58% yield).
LCMS: [M+H]⁺ = 381.1
¹H NMR (400 MHz, DMSO-*d₆*) *δ* 8.65 (s, 1H), 7.60 (dd, *J* = 7.6, 1.2 Hz, 1H), 7.45 (dd,*J* = 7.6, 1.2 Hz, 1H), 7.22 (td, *J =* 7.6, 1.2 Hz, 1H), 7.12 (td, *J =* 7.6, 1.2 Hz, 1H), 6.53 (s, 2H), 4.75 (s, 2H), 4.06 - 3.94 (m, 2H), 2.95 - 2.88 (m, 2H), 2.12 (s, 2H), 2.02 (s, 6H), 1.11 (s, 3H), 0.50 (d, *J =* 1.2 Hz, 2H), 0.28 (d, *J =* 1.6 Hz, 2H).

### Example A1 Potassium Ion Channel Opener Activity Test (FDSS/µ CELL Detection)

### 1. Experimental Method:

### 1.1 Experimental Procedure

Cell preparation: CHO-KCNQ2 cells were cultured in a 175 cm² culture flask, and when the cell density grew to 60~80%, the culture medium was removed and the cells were washed once with 7 mL PBS (Phosphate Buffered Saline), and then 3 mL of 0.25% Trypsin was added for digestion. After completion of digestion, 7 mL of culture medium (90% DMEM/F12 + 10% FBS + 500 µg/mL G418) was added to neutralize, and centrifuged at 800 rpm for 3 min; the supernatant was aspirated, then 5 mL of culture medium was added to resuspend the cells, and the cells were counted.

Cell spreading: according to the cell counting results, the density was adjusted to 3×10⁴ cells/well. After standing at room temperature for 30 minutes, the cells were placed in a CO₂ incubator at 37°C overnight, and incubated for 16-18 hours to reach a cell density of about 80%.

Fluorescent dye incubation: Cell culture solution was discarded, 80 µL/well of loading buffer was added and the cells were incubated for 60 minutes at room temperature and protected from light.

Compound incubation: Loading buffer was discarded, 80 µL/well of prepared compound solution was added, and the cells were incubated for 20 min at room temperature and protected from light.

Fluorescence data acquisition: Real-time fluorescence signal recording was performed using an FDSS/µCELL instrument with an excitation wavelength of 480 nm and an emission wavelength of 540 nm, and the signal was recorded once per second. 10 seconds of the baseline was recorded, then 20 µL/well of stimulation buffer was added, and the recording was continued until the end of 180 seconds.

### 1.2 Solution Preparation

Loading buffer: 10mL/plate, prepared as follows:

| Component | Volume |
|---|---|
| PowerLoad^{™} concentrate, 100X (component C) | 100 µL |
| FluxOR^{™} reagent, reconstructed in DMSO (step 1.2) | 10 µL |
| Deionized water | 8.8 mL |
| FluxOR^{™} test buffer, 10X (component B) | 1 mL |
| probenecid, reconstructed in deionized water (step 1.1) | 100 µL |
| Total Volume | 10mL |

Test buffer: 100mL/plate, prepared as follows:

| Component | Volume |
|---|---|
| Deionized water | 8.9 mL |
| FluxOR^{™} test buffer, 10X (component B) | 1 mL |
| probenecid, reconstructed in deionized water (step 1.1) | 100 µL |
| Total Volume | 10 mL |

Stimulation buffer: 5 mL/plate, prepared as follows:

| Component | Volume | |
|---|---|---|
| | + K⁺ | - K⁺ |
| Deionized water | 2.5 mL | 3.5 mL |
| FluxOR^{™} Chlorine Free Buffer, 5X (Component E) | 1 mL | 1 mL |
| K₂SO₄ Concentrate (125 mM concentrated solution of K₂SO₄, Component F) | 1 mL | / |
| Tl₂SO₄ concentrate (50 mM concentrated solution of Tl₂SO₄, component G) | 0.5 mL | 0.5 mL |
| Total Volume | 5 mL | 5 mL |

The above buffers were obtained from a commercially available kit called FluxOR potassium ion channel assay.

### 1.3 Compound preparation

20 mM stock solution of compound in DMSO was prepared, 10 µL of 20 mM compound stock solution was taken into 20 µL of DMSO solution, and a 3-fold serial dilution was performed to form 8 intermediate concentrations; then the intermediate concentrations of the compounds were taken respectively into test buffer, and a 200-fold dilution was performed to obtain the final concentration to be tested, and 80 µL was taken and added to the assay plate.

The highest test concentration was 100 µM, and there was a total of 8 concentrations, i.e., 100, 33.33, 11.11, 3.70, 1.23, 0.41, 0.137, 0.045µM, respectively, with 3 replicate wells for each concentration.

The amount of DMSO in the final test concentration did not exceed 0.5%, and this concentration of DMSO had no effect on KCNQ2 potassium channels.

### 1.4 Data analysis

The experimental data were analyzed by Excel 2007, GraphPad Prism 5.0 software, and the ratio of 180 s was counted to calculate the agonistic effect. The agonistic effect of the compound was calculated by the following equation: $\begin{matrix}Percent of \\ agonism\end{matrix} = \frac{\begin{matrix}Ratio of fluorescence signal with compound - Ratio of \\ fluorescence signal without compound\end{matrix}}{Ratio of fluorescence signal without compound} \times 100 %$

### 1.5 Quality Control

Environment: Temperature ~25 °C
Reagent: FluxORTM detection kit (Invitrogen, Cat # F0017)

### The experimental data in the report must meet the following criteria: Z' Factor > 0.5

2. Results: See Table 1 for details, wherein a smaller EC₅₀ indicates a higher activity of the corresponding compound.

**Table 1. Test results of some of the compounds of the present invention**

| Compound | Maximum agonistic rate (%) | EC₅₀(uM) |
|---|---|---|
| 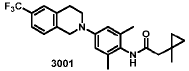 | 45.61 | 0.086 |
| 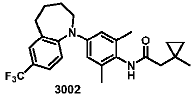 | 28.10 | 0.58 |
| 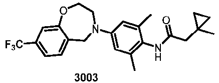 | 39.57 | 0.018 |
| 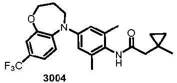 | 17.67 | 0.58 |
| 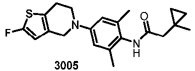 | 46.68 | 0.11 |
| 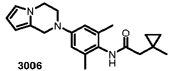 | 34.86 | 0.27 |
| 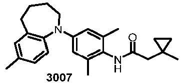 | 41.54 | 0.030 |
| 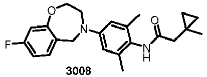 | 40.87 | 0.017 |
| 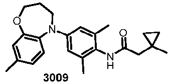 | 36.07 | 0.071 |
| 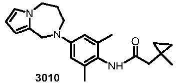 | 35.32 | 3.70 |
| 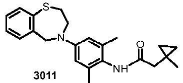 | 49.39 | 0.0043 |

### Reference for the above test method:

Zhaobing Gao et al, Journal of Biological Chemistry. 2010, 285(36): 28322-28332.

All documents referred to in the present invention are incorporated by reference herein as if each document is individually incorporated by reference. Further, it should be understood that upon reading the above teachings of the present invention, various modifications or changes may be made to the present invention by those skilled in the art, and those equivalents also fall within the scope defined by the appended claims of the present application.

## Claims

1. A compound of formula I, or a pharmaceutically acceptable salt thereof, 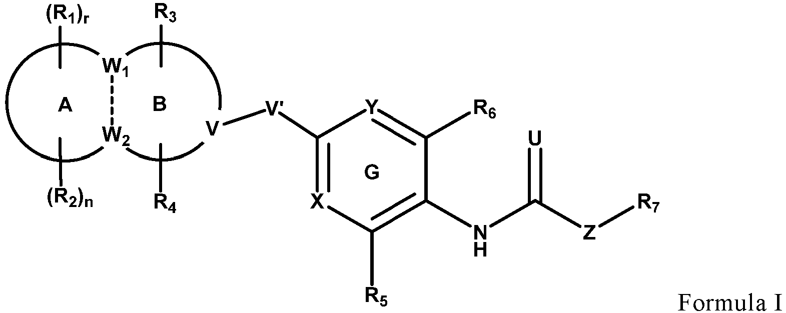 wherein,
ring A is selected from the group consisting of: none, C₆₋₁₀ aryl, 4-7-membered heteroaryl containing 1-3 heteroatoms selected from N, O and S, saturated or unsaturated C₃₋₆ cyclic hydrocarbon group, and 3-10-membered heterocyclyl containing 1-3 heteroatoms selected from N, O and S;
R₁, R₂, R₃ and R₄ are independently selected from the substituted or unsubstituted group consisting of: hydrogen, deuterium, halogen, cyano, -OH, -COOH, nitro, C₁₋₆ alkyl, C₃₋₆ cycloalkyl, C₁₋₆ alkoxy, C₃₋₆ cycloalkyloxy, C₂₋₆ alkenyl, C₂₋₆ alkynyl, saturated or unsaturated C₃₋₆ cyclic hydrocarbon group, 3-10-membered heterocyclyl containing 1-3 heteroatoms selected from N, O and S, C₆₋₁₀ aryl, 5-14-membered heteroaryl containing 1-3 heteroatoms selected from N, O and S, C₆₋₁₂ arylalkyl, -N(R₁')(R₂'), -C(O)-R₁', -C(O)-N(R₁') (R₂'), -C(O)-OR₁', -N(R₁')-C(O)-R₂', -S(O)ₘ-R₁', -S(O)ₘ-N(R₁')(R₂'), -S(O)ₘ-OR₁', and -N(R₁')-S(O)ₘ-R₂', and the "substituted" refers to being substituted by one or more substituents selected from the group consisting of: halogen, C₁₋₆ alkyl, C₃₋₆ cycloalkyl, C₁₋₆ alkoxy, C₃₋₆ cycloalkyloxy, C₁₋₆ haloalkyl, C₃₋₆ halocycloalkyl, C₁₋₆ haloalkoxy and C₃₋₆ halocycloalkyloxy;
n and r are independently selected from the group consisting of: 0, 1 and 2;
R₁' and R₂' are independently selected from the group consisting of: hydrogen, C₁₋₆ alkyl, C₃₋₆ cycloalkyl, or R₁' and R₂' together with the attached N-atom form a saturated or unsaturated 3-10-membered heterocyclyl containing 1-3 heteroatoms selected from N, O and S; and the above alkyl, cycloalkyl and heterocyclyl are optionally substituted by one or more substituents selected from the group consisting of: =O, halogen, C₁₋₆ alkyl and C₃₋₆ cycloalkyl;
m is selected from the group consisting of: 1 and 2;
W₁ and W₂ are independently selected from the group consisting of: none, C and N;
and W₁ and W₂ are not simultaneously none;
------ is selected from the group consisting of: none, single bond and double bond;
ring B is selected from the group consisting of: C₆₋₁₀ aryl, 4-7-membered heteroaryl containing 1-3 heteroatoms selected from N, O and S, saturated or unsaturated C₃₋₁₀ cyclic hydrocarbon group, 3-10-membered heterocyclyl containing 1-3 heteroatoms selected from N, O and S, 3-10-membered bridged heterocyclyl containing 1-3 heteroatoms selected from N, O and S;
V is selected from the group consisting of: C, CRs and N;
R₈ is selected from the group consisting of: hydrogen, C₁₋₆ alkyl, C₃₋₆ cycloalkyl, C₂₋₆ alkenyl and C₂₋₆ alkynyl; and the above alkyl and cycloalkyl are optionally substituted by one or more substituents selected from the group consisting of halogen, C₁₋₆ alkyl and C₃₋₆ cycloalkyl;
V' is selected from the group consisting of: -(CH₂)ₚ-, -C(CH₃)₂-, 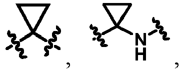 -NH-, -(CH₂)ₚ-NH-, -CF₂-NH-, -C(CH₃)₂-NH- and 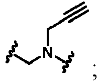 p is selected from the group consisting of: 0, 1 and 2;
X and Y are independently selected from the group consisting of: N and CR₉;
R₉ is selected from the group consisting of: hydrogen, halogen, cyano, amino, hydroxyl, C₁₋₆ alkyl, C₃₋₆ cycloalkyl and C₁₋₆ alkoxy; and the above amino, alkyl, cycloalkyl and alkoxy are optionally substituted by one or more substituents selected from the group consisting of halogen, C₁₋₆ alkyl, and C₃₋₆ cycloalkyl;
R₅ and R₆ are independently selected from the group consisting of: hydrogen, halogen, cyano, amino, hydroxyl, C₁₋₆ alkyl, C₃₋₆ cycloalkyl and C₁₋₆ alkoxy; and the above amino, alkyl, cycloalkyl and alkoxy are optionally substituted by one or more substituents selected from the group consisting of halogen, C₁₋₆ alkyl and C₃₋₆ cycloalkyl;
U is selected from the group consisting of: O, S and N(R₁₀);
Z is selected from the group consisting of: O, -(CH₂)_{q}- and -N(R₁₁)-;
q is selected from the group consisting of: 0, 1 and 2;
R₁₀ and R₁₁ are independently selected from the group consisting of: hydrogen, C₁₋₆ alkyl and C₃₋₆ cycloalkyl; and the above alkyl and cycloalkyl are optionally substituted by one or more substituents selected from the group consisting of halogen, C₁₋₆ alkyl and C₃₋₆ cycloalkyl;
R₇ is selected from the group consisting of: C₁₋₆ alkyl, C₃₋₆ cycloalkyl, C₅₋₈ bridged cyclic group, adamantyl, C₆₋₁₀ aryl, 3-10-membered heteroaryl containing 1-3 heteroatoms selected from N, O and S, 4-8-membered heterocycloalkyl containing 1-3 heteroatoms selected from N, O and S, C₃₋₆ cycloalkenyl, C₂₋₆ alkenyl and C₂₋₆ alkynyl; and the above alkyl, cycloalkyl, bridged cyclic group, adamantyl, aryl, heteroaryl, heterocycloalkyl, cycloalkenyl, alkenyl, and alkynyl are optionally substituted by one or more substituents selected from the group consisting of: hydrogen, halogen, cyano, nitro, amino, hydroxyl, C₁₋₆ alkyl-CO-, C₁₋₆ alkyl, C₃₋₆ cycloalkyl, C₆₋₁₀ aryl, C₁₋₆ haloalkyl, C₁₋₆ alkoxy, C₁₋₆ alkylamino and C₁₋₆ haloalkoxy.

2. The compound according to claim 1, or a pharmaceutically acceptable salt thereof, wherein,
ring A is selected from the group consisting of: none, C₆₋₁₀ aryl and 4-7-membered heteroaryl containing 1-3 heteroatoms selected from N, O and S;
R₁, R₂, R₃ and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n and r are selected from the group consisting of: 0, 1 and 2;
W₁ and W₂ are independently selected from the group consisting of: none, C and N;
and W₁ and W₂ are not simultaneously N;
ring B is selected from the group consisting of: C₆₋₁₀ aryl, 4-7-membered heteroaryl containing 1-3 heteroatoms selected from N, O and S, saturated or unsaturated C₃₋₁₀ cyclic hydrocarbon group, 3-10-membered heterocyclyl containing 1-3 heteroatoms selected from N, O and S, 3-10-membered bridged heterocyclyl containing 1-3 heteroatoms selected from N, O and S;
VisN;
V' is selected from the group consisting of: -(CH₂)ₚ-, -NH- and -CH₂-NH-;
p is selected from the group consisting of 0 and 1;
X and Y are CH;
R₅ and R₆ are independently selected from the group consisting of: halogen and C₁₋₆alkyl; and the above alkyl is optionally substituted by one or more substituents selected from halogen;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

3. The compound according to claim 1, or a pharmaceutically acceptable salt thereof, wherein, 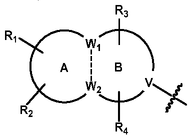 in the formula I is selected from the group consisting of: 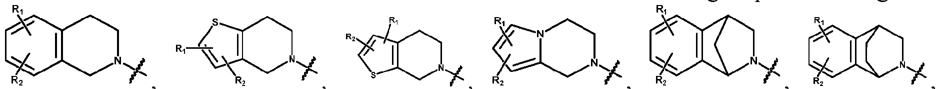 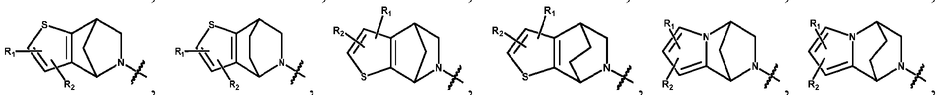 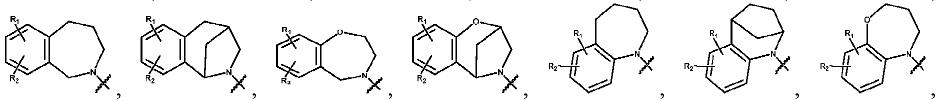 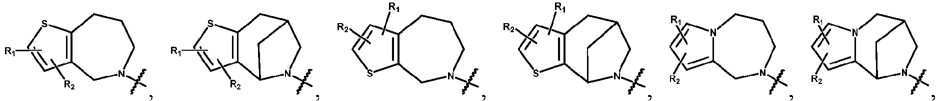 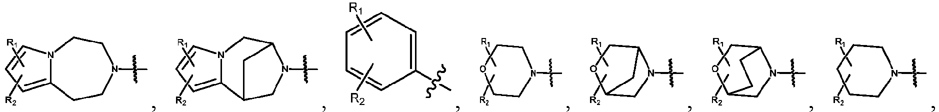 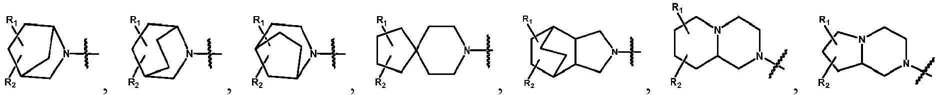 and 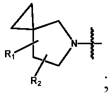
R₁, R₂, R₃ and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n and r are independently selected from the group consisting of: 0, 1 and 2.

4. The compound according to claim 1, or a pharmaceutically acceptable salt thereof, wherein,
R₇ is selected from the group consisting of: C₃₋₆ cycloalkyl and C₅₋₈ bridged cyclic group.

5. The compound according to claim 1, or a pharmaceutically acceptable salt thereof, wherein, 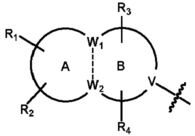 in the formula I is selected from the group consisting of: 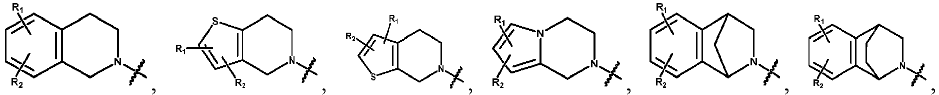 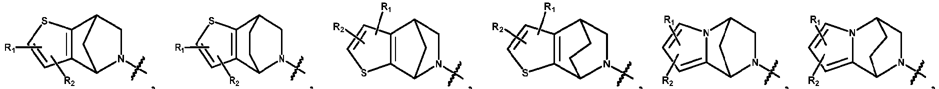 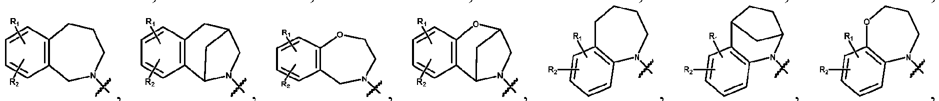 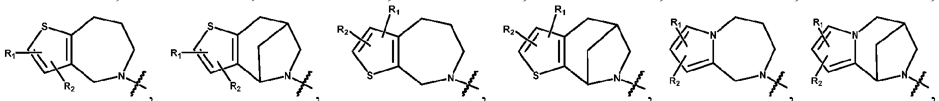 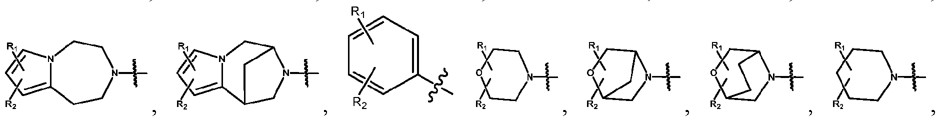 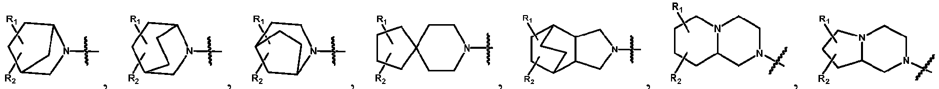 and 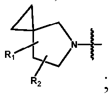
R₁, R₂, R₃ and R₄ are independently selected from the substituted or unsubstituted group consisting of: hydrogen, halogen, C₁₋₆alkyl and C₁₋₆alkoxy; and the "substituted" refers to being substituted by one or more substituents selected from halogen;
n and r are independently selected from the group consisting of: 0, 1 and 2;
V' is selected from the group consisting of: -(CH₂)ₚ-, -NH-, -CH₂-NH- and 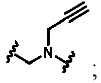
p is selected from the group consisting of 0 and 1;
X and Y are independently selected from the group consisting of: N and CH;
R₅ and R₆ are independently selected from the group consisting of: hydrogen, halogen, amino, C₁₋₆alkyl and C₁₋₆alkoxy;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

6. The compound according to claim 5, or a pharmaceutically acceptable salt thereof, wherein,
R₁, R₂, R₃, and R₄ are independently selected from the substituted or unsubstituted group consisting of: hydrogen, halogen and C₁₋₆alkyl;
n is selected from the group consisting of: 0, 1 and 2;
V' is selected from the group consisting of: -(CH₂)ₚ-, -NH- and -CH₂-NH-;
p is selected from the group consisting of 0 and 1;
X and Y are CH;
R₅ and R₆ are independently selected from the group consisting of: halogen and C₁₋₆alkyl;
U is O;
Z is CH₂;
R₇ is selected from the group consisting of: C₃₋₆ cycloalkyl and C₅₋₈ bridged cyclic group; and the above cycloalkyl and bridged cyclic group are optionally substituted by one or more substituents selected from the group consisting of: hydrogen, halogen, cyano, C₁₋₆alkyl and C₁₋₆haloalkyl.

7. The compound according to claim 1, or a pharmaceutically acceptable salt thereof, wherein, the compound is selected from the group consisting of:

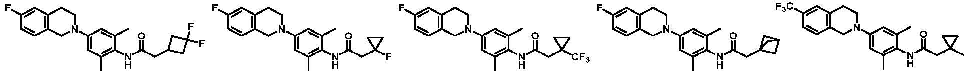

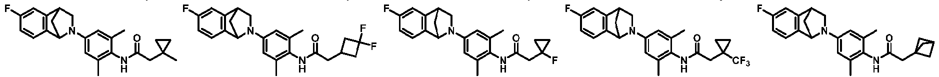

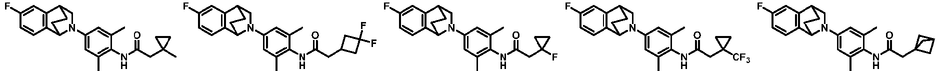

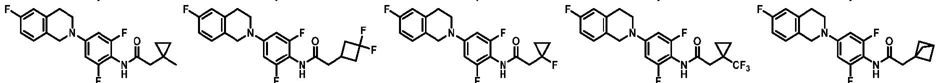

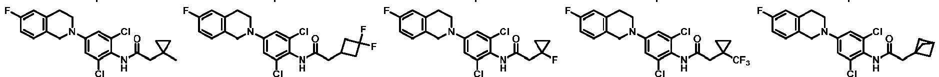

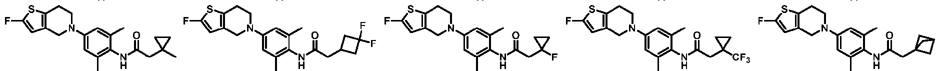

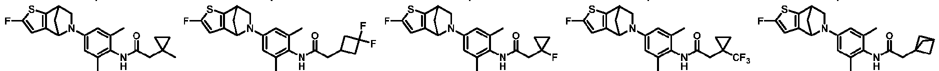

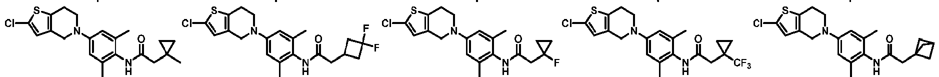

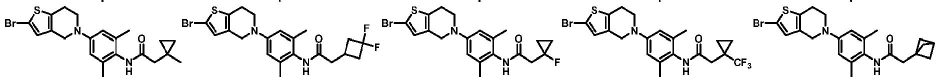

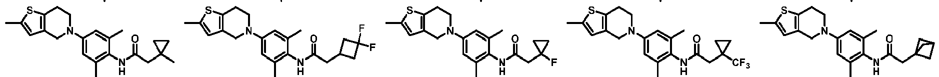

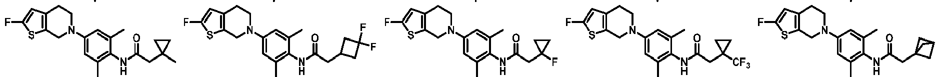

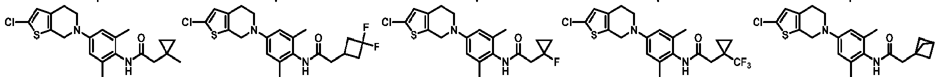

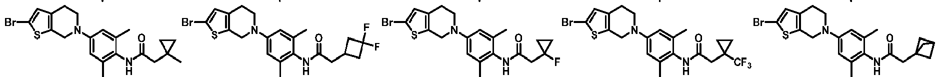

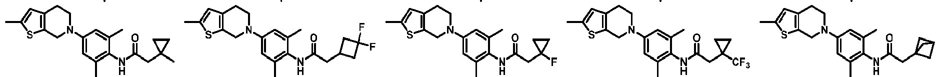

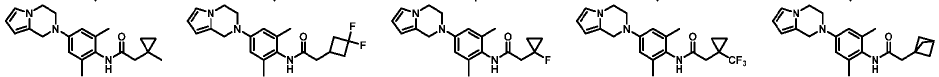

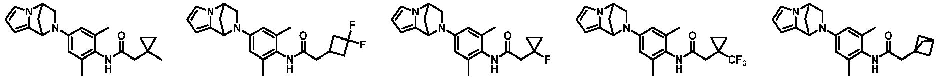

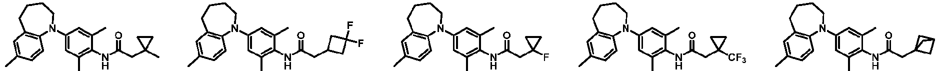

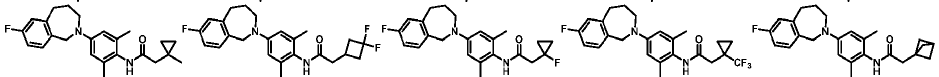

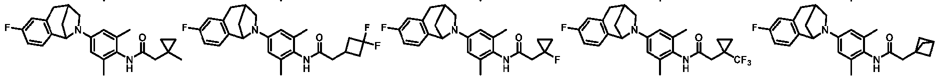

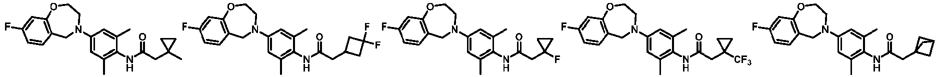

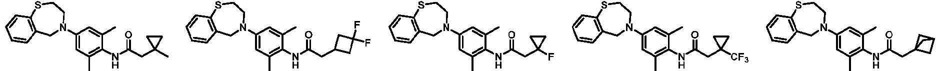

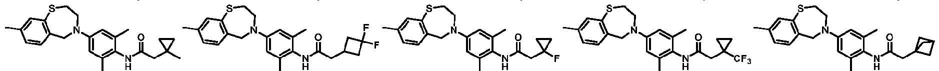

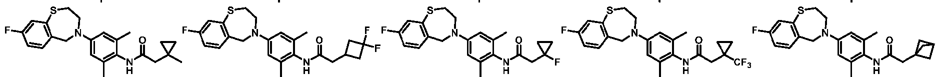

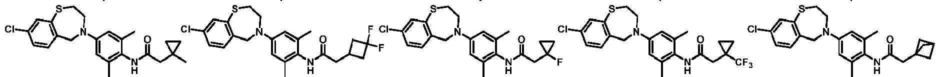

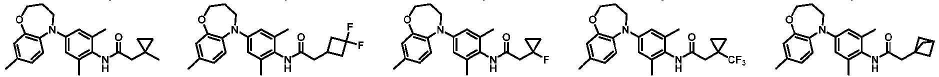

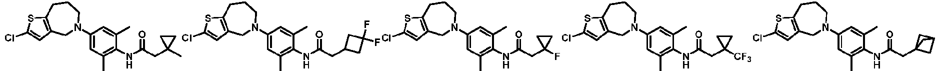

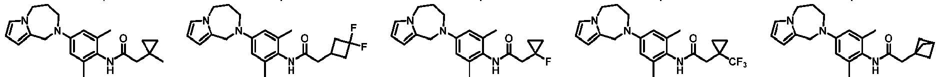

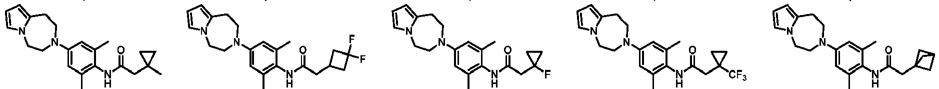

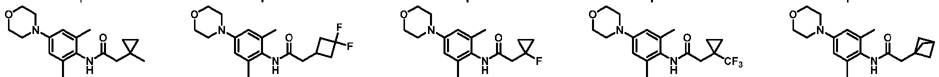

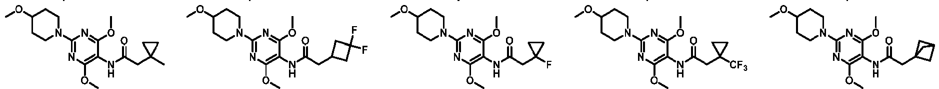

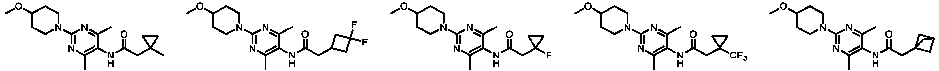

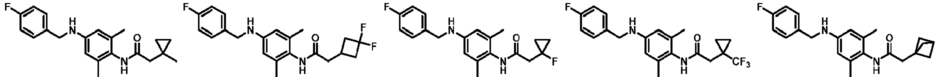

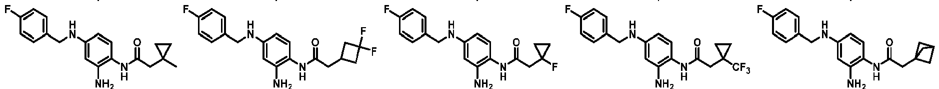

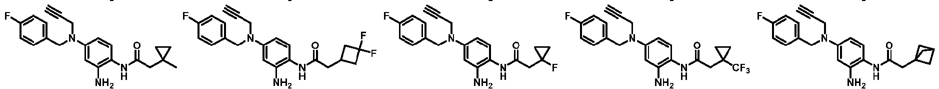

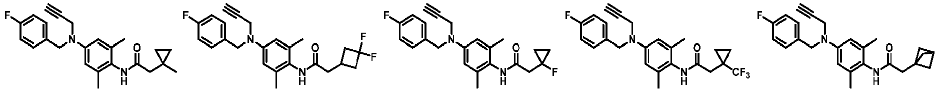

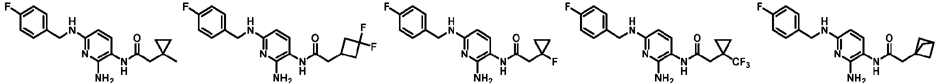

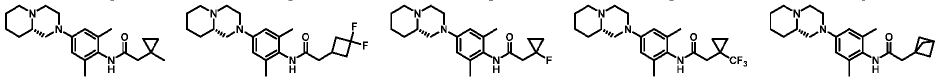

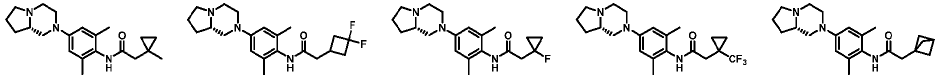

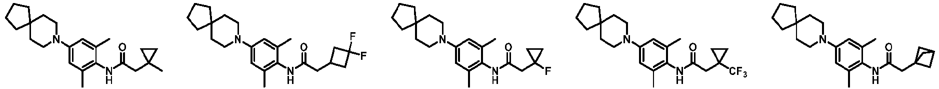

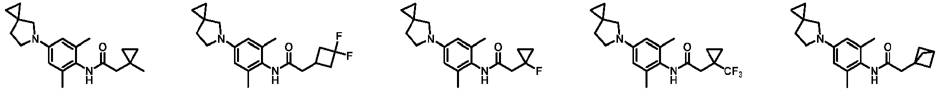

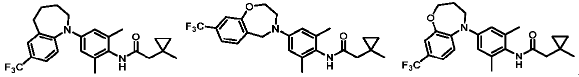

8. A pharmaceutical composition comprising one or more pharmaceutically acceptable carriers and a therapeutically effective amount of one or more of the compounds according to claim 1 or a pharmaceutically acceptable salt thereof.

9. A use of the compound according to claim 1 or a pharmaceutically acceptable salt thereof in the preparation of a drug for the prevention and/or treatment of a disease sensitive to potassium ion channels.

10. The use according to claim 9, wherein the disease sensitive to potassium ion channels is a central nervous system disease.
